(19) Europäisches Patentamt
European Patent Office
Office européen des brevets

(11) Veröffentlichungsnummer : **0 096 004**
**B1**

# (12) EUROPÄISCHE PATENTSCHRIFT

(45) Veröffentlichungstag der Patentschrift : **26.04.89**

(21) Anmeldenummer : **83810214.3**

(22) Anmeldetag : **20.05.83**

(51) Int. Cl.⁴ : **C 07 D403/12**, C 07 D239/42, A 01 N 47/36 // (C07D239/42, 237:18, 241:18)

## (54) Neue Sulfonyl(thio)harnstoffe, Verfahren zu deren Herstellung und deren Verwendung als Herbizide und/oder Wachstumsregulatoren.

(30) Priorität : **28.05.82 CH 3314/82**

(43) Veröffentlichungstag der Anmeldung : **07.12.83 Patentblatt 83/49**

(45) Bekanntmachung des Hinweises auf die Patenterteilung : **26.04.89 Patentblatt 89/17**

(84) Benannte Vertragsstaaten : **AT BE CH DE FR GB IT LI NL**

(56) Entgegenhaltungen :
**EP-A- 0 013 480**
**EP-A- 0 035 893**
**CHEMICAL ABSTRACTS, Band 97, 1982, Seite 45, Nr. 33372d, Columbus, Ohio, USA; & JP - A - 82 07129 (DAIICHI SEIYAKU CO., LTD.) 09.02.1982**
**CHEMICAL ABSTRACTS, Band 79, 1973, Seite 438, Nr. 92175t, Columbus, Ohio, USA; M. HATTORI et al.: "Synthesis of some 2H-pyrazino[2,3-e][1,2,4]thiadiazine 1,1-dioxides" & BULL. CHEM. SOC. JAP. 1973, 46(6), 1890-1891**
**CHEMICAL ABSTRACTS, Band 77, 1972, Seite 17, Nr. 13938b, Columbus, Ohio, USA; F.P. MEYER: "Interaction between proteins and sulfanilamides" & ACTA BIOL. MED. GER. 1972, 28(1), 21-25**
**THE JOURNAL OF ORGANIC CHEMISTRY, Band 44, Nr. 26, 1979, Seiten 4970-4971, Columbus, Ohio, USA; R.B. SILVERMAN et al.: "A mild method of hydrolysis of 2,4-dialkoxy-6-substituted pyrimidines to 6-substituted uracils"**
**CHEMICAL ABSTRACTS, Band 67, 1967, Seite 7520, Nr. 79902k, Columbus, Ohio, USA; T.C. EICKHOFF et al.: "In vitro activity of carbonic anhydrase inhibitors against Neisseria meningitidis" & ANTIMICROB. AGENTS CHEMOTHER. 1966, 389-392**

(73) Patentinhaber : **CIBA-GEIGY AG**
**Klybeckstrasse 141**
**CH-4002 Basel (CH)**

(72) Erfinder : **Böhner, Beat, Dr.**
**Hügelweg 3**
**CH-4102 Binningen (CH)**
Erfinder : **Föry, Werner, Dr.**
**Benkenstrasse 65**
**CH-4054 Basel (CH)**
Erfinder : **Schurter, Rolf, Dr.**
**Holzmattstrasse 45**
**CH-4102 Binningen (CH)**
Erfinder : **Pissiotas, Georg, Dr.**
**Breslauerstrasse 8**
**D-7850 Lörrach (DE)**

**EP 0 096 004 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ANNALES PHARMACEUTIQUES FRANCAISES, Band 24, Nr. 9-10, 1966, Seiten 593-605; J.L. KIGER et al.: "Procédés de diagnose différentielle rapide en série des dérivés sulfamidés usuels"
JOURNAL OF THE CHEMICAL SOCIETY PERKIN I, Transactions I, 1972, Seiten 522-527, London, GB; D.J. BROWN et al.: "Simple pyrimidines. Part XIV. The formation and reactions of some derivatives of simple pyrimidinesulphonic acids"
Die Akte enthält technische Angaben, die nach dem Eingang der Anmeldung eingereicht wurden und die nicht in dieser Patentschrift enthalten sind.

**Beschreibung**

Die vorliegende Erfindung umfasst eine Gruppe neuer Sulfonyl(thio) harnstoffe, die wertvolle pflanzenwachstumsregulierende Eigenschaften aufweisen und sich zur gezielten Beeinflussung des Pflanzenwuchses, zur Bekämpfung von Unkräutern z. B. in Kulturen von Nutzpflanzen und/oder zur Beeinflussung der phytotoxischen Eigenschaften anderer herbizider Substanzen eignen. Sie umfasst ferner die Herstellung dieser Sulfonyl(thio)harnstoffe, sowie landwirtschaftliche Mittel, die diese Substanzen als Wirkstoff enthalten, und zu ihrer Herstellung benötigte neue Zwischenprodukte.

Es werden hierin Verbindungen der Formel I

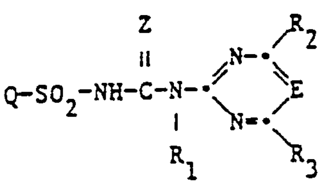

(I)

worin

$R_1$ für Wasserstoff oder $C_1$-$C_5$-Alkyl steht ;

$R_2$ und $R_3$ unabhängig voneinander Wasserstoff, $C_1$-$C_5$-Alkyl, $C_1$-$C_5$-Alkoxy, $C_1$-$C_5$-Alkylthio, $C_1$-$C_5$-Halogenalkyl, Halogen, $C_1$-$C_5$-Halogenalkylthio $C_1$-$C_4$-Alkylamino, Di-$C_1$-$C_4$-alkylamino, eine Alkoxyalkylgruppe oder Alkoxyalkoxygruppe mit maximal 6 Kohlenstoffatomen bedeuten ;

Z für Sauerstoff oder Schwefel steht ;

E für —CH= oder —N= steht und

Q einen über ein Kohlenstoffatom gebundenen, gegebenenfalls durch Halogen, Pseudohalogen, Nitro, $C_1$-$C_6$-Alkyl, $C_1$-$C_6$-Halogenalkyl, $C_1$-$C_6$-Alkoxy, $C_1$-$C_6$-Alkylthio, $C_1$-$C_6$-Halogenalkoxy, $C_1$-$C_6$-Halogenalkylthio, Amino, $C_1$-$C_6$-Alkylamino, Di-$C_1$-$C_6$-alkylamino, $C_1$-$C_6$-Alkylcarbonylamino, $C_1$-$C_6$-Alkylcarbonyl, $C_1$-$C_6$-Alkoxycarbonyl, $C_1$-$C_6$-Alkylthiocarbonyl, Carbamoyl, $C_1$-$C_6$-Alkylaminocarbonyl, Di-$C_1$-$C_6$-alkylaminocarbonyl, $C_1$-$C_6$-Alkylsulfinyl, $C_1$-$C_6$-Alkylsulfonyl, $C_2$-$C_6$-Alkenyloxy, $C_3$-$C_6$-Alkinyloxy oder ein gegebenenfalls durch Halogen, Nitro, Cyano, $C_1$-$C_6$-Alkyl, $C_1$-$C_6$-Alkoxy, $C_1$-$C_6$-Halogenalkyl, $C_1$-$C_6$-Halogenalkoxy substituiertes Phenyl, Phenoxy, Phenylthio oder eine gegebenenfalls durch Halogen und/oder $C_1$-$C_6$-Alkyl substituierte Benzylgruppe substituierten Pyrimidin-, Pyridazin-, Pyrazin- oder Triazin-Rest bedeutet ; unter Einschluss ihrer Salze. Der heterocyclische Rest Q kann unsubstituiert, ein- oder mehrfach substituiert sein, und ist vorzugsweise Pyrazin. Bevorzugt sind dabei Substituenten, deren aliphatischer Anteil maximal 4 Kohlenstoffatome aufweist.

Unter Alkyl oder Alkylanteil eines anderen Substituenten sind je nach Zahl der angegebenen Kohlenstoffatome beispielsweise folgende Gruppen zu verstehen : Methyl, Aethyl, Propyl, Butyl oder Pentyl sowie ihre Isomeren wie Isopropyl, Isobutyl, tert.-Butyl, Isopentyl usw. Halogenalkyl steht für eine ein- oder mehrfach durch Halogen substituierten Alkylrest, beispielsweise für $CF_3$, $CH_2Cl$, $CCl_3$, $CHCl_2$, $CH_2J$, $C_2H_4Cl$, $C_2H_4Br$, $CH_2Br$ usw. Halogen steht hier und im folgenden für Fluor, Chlor, Brom oder Jod, vorzugsweise für Fluor, Chlor oder Brom. Die auf dem Agrargebiet einsetzbaren Salze, sind z. B. diejenigen, die die Verbindungen der Formel I mit Aminen, Alkali- und Erdalkalimetallbasen oder mit quaternären Ammoniumbasen bilden. Hierbei sind unter Alkali- oder Erdalkalibasen insbesondere die Hydroxide wie Lithium, Natrium, Kalium, Magnesium oder Calcium zu verstehen.

Beispiele für zur Salz bildung geeignete Amine sind primäre, sekundäre und tertiäre aliphatische und aromatische Amine wie Methylamin, Aethylamin, Propylamin, i-Propylamin, die vier isomere Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z. B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Pyridinylsulfonylharnstoffe mit herbizider und pflanzenwuchsregulierender Wirkung sind aus EP-A-13 480 und EP-A-35 893 bekannt.

Die Verbindungen der Formel I sind bei Raumtemperatur stabile/biologisch aktive Oele, Harz oder Feststoffe, die sich durch sehr wertvolle pflanzenwachstumsregulierende, vor allem wachstumshemmende Eigenschaften auszeichnen. Sie lassen sich daher auf dem Agrarsektor oder auf verwandten Anwendungsgebieten zur gezielten Reduktion des Wachstums monokotyler und dikotyler Pflanzen einsetzen, wobei sie je nach Art der Verwendung bzw. der Aufwandmenge selektiv herbizide bis totalherbizide Eigenschaften entfalten.

Zu einer bevorzugten Gruppe von Verbindungen der Formel I gehören diejenigen, worin Z Sauerstoff bedeutet und $R_1$ für Wasserstoff steht.

Eine besonders bevorzugte Gruppe von Wirkstoffen besteht aus Verbindungen der Formel I, worin $R_1$ für Wasserstoff steht, $R_2$ und $R_3$ unabhängig voneinander $C_1$-$C_3$-Alkyl, $C_1$-$C_3$-Alkoxy, $C_1$-$C_3$-Halogenalkyl, $C_1$-$C_3$-Halogenalkoxy, Halogen oder $C_2$-$C_4$-Alkoxyalkyl, Z Sauerstoff, E Stickstoff oder CH bedeuten, Q für einen unter Formel I definierten heterocyclischen Rest steht, der gegebenenfalls durch Chlor, Brom, Fluor, Nitro, Cyano, $C_1$-$C_4$-Alkylcarbonyl, $C_1$-$C_4$-Alkoxycarbonyl, $C_1$-$C_3$-Alkoxy, $C_1$-$C_3$-Alkylthio, $C_3$-$C_5$-Alkoxyalkoxy, $C_1$-$C_3$-Alkylsulfinyl, $C_1$-$C_3$-Alkylsulfonyl, $C_1$-$C_4$-Alkyl, Trifluormethyl, Trichlormethyl, Cyano-$C_1$-$C_4$-Alkyl, $C_2$-$C_4$-Alkenyloxy, $C_3$-$C_4$-Alkinyloxy oder N-$C_1$-$C_4$-Alkylcarbonylamino substituiert ist.

Folgende Einzelsubstanzen sind besonders bevorzugt:

N-(2-Methyl-pyrazin-3-ylsulfonyl)-N'-(4-methoxy-6-methyl-1,3,5-triazin-2-yl)-harnstoff und
N-(2-Methoxy-pyrazin-3-ylsulfonyl)-N'-(4-methoxy-6-methyl-1,3,5-triazin-2-yl)-harnstoff.

Die Wirkstoffe der Formel I können in an sich bekannter Weise in einem reaktionsinerten, organischen Lösungsmittel oder Lösungsmittelgemisch hergestellt werden.

Man kann dabei im einzelnen folgendermassen vorgehen und Verbindungen der Formel I, gemäss Gleichung [1],

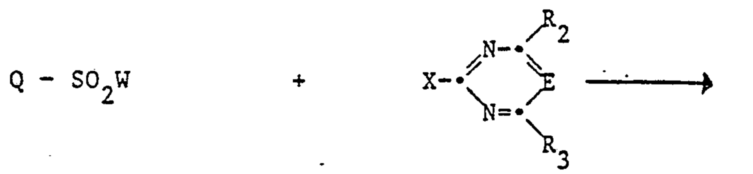

(I) (1)

(II) (III)

dadurch herstellen, dass man ein Phenylsulfonylderivat II mit einer Verbindung der Formel III umsetzt, wobei X und W für die Gruppen $-NH_2$, $-N=C=Z$ oder $-NR_1-CZ-OR$ stehen; die Substituenten $R_1$, $R_2$, $R_3$, Q, Z und E die unter Formel I angegebenen Bedeutungen haben, R für einen aliphatischen oder aromatischen Rest, vorzugsweise für $C_1$-$C_4$-Alkyl, Phenyl oder Benzyl steht; mit der Massgabe, dass die Reaktionspartner der Formeln II und III stets so gewählt werden, dass eine Aminofunktion entweder mit einer Isocyanato- bzw. Isothiocyanatofunktion oder mit der $[-NR_1-CZ-OR]$-gruppe zur Reaktion gelangt.

Das Verfahren wird vorteilhafterweise in Gegenwart einer Base durchgeführt. In manchen Fällen kann es von Vorteil sein, wenn man unter Schutzgasatmosphäre z. B. unter Stickstoff arbeitet.

Das Herstellungsverfahren ist Bestandteil vorliegender Erfindung.

Die erhaltenen Verfahrens-Produkte der Formel I können gewünschtenfalls durch Reaktion mit Aminen, Alkalimetall- oder Erdalkalimetallhydroxiden oder quaternären Ammoniumbasen in basischen, landwirtschaftlich verwendbare Additionssalze übergeführt werden. Dieses geschieht beispielsweise durch Umsetzen mit der äquimolaren Menge Base und Verdampfen des Lösungsmittels. Solche Umsetzungen wie auch die Herstellung einzelner Ausgangsstoffe der Formel III sind bekannt und beispielsweise in den US Patentschriften US-A-3 384 757 und 3 410 887 beschrieben.

Die Ausgangsverbindungen der Formel III sind bekannt oder werden nach an sich bekannten Methoden hergestellt.

Die Verbindungen der Formel II, in denen W für $NH_2$ steht, sind teilweise bekannt. So sind aus folgenden Literaturstellen: J. Org. Chem. 44 (1979) 4970-4971; Chem. Abstr. 79 (1973) 92175t; Chem. Abstr. 97 (1982) 33372d und J.C.S. Perkin I, Trans I, 1972, 522-527 diejenigen Sulfonamide bekannt, worin Q für 4-Methyl-2-pyrimidinyl, 5-Methyl-2-pyrimidinyl, 2,4-Dimethoxy-6-pyrimidinyl, 4-Methoxy-6-pyrimidinyl, 3-Amino-2-pyrazinyl, 2-Amino-5-methyl-3-pyrazinyl, 3-Amino-5,6-dimethyl-2-pyrazinyl oder 4,6-Dimethyl-2-pyrimidinyl steht. Die neuen Verbindungen der Formel II sind speziell für die Synthese der Wirkstoffe der Formel I hergestellt worden. Sie sind daher Bestandteil vorliegender Erfindung.

Die Ausgangsverbindungen der Formel II, in denen W für $-NH-CZ-OR$ steht, sind neu und speziell für die Synthese der Verbindungen der Formel I entwickelt worden. Sie bilden daher einen weiteren Aspekt der vorliegenden Erfindung. Ihre Herstellung erfolgt nach an sich bekannten Methoden.

Die Sulfonamide der Formel II können durch Umsetzen der entsprechenden Sulfonylchloride oder -fluoride mit Ammoniak oder Ammoniumhydroxid-Lösungen gewonnen werden, siehe z. B. J. Chem. Soc. Perkin Trans. 1, 1972, 522.

Die Isocyanate der Formeln II und III können durch Umsetzung der zugrundeliegenden Amino-Verbindungen mit Phosgen in Anwesenheit von Butylisocyanat in einem chlorierten Kohlenwasserstoff als Lösungsmittel bei Rückflusstemperatur erhalten werden. Siehe dazu « Neuere Methoden der präparativen organischen Chemie » Band VI, 211-229, Verlag Chemie, Weinheim, 1970. Bei Verwendung von Thiophosgen gelangt man auf analoge Weise zur Isothiocyanaten II und III.

Die Isothiocyanate der Formel II werden darüberhinaus auch durch Behandlung der Sulfonamide II mit Schwefelkohlenstoff und Kaliumhydroxyd und anschliessender Umsetzung des Dikaliumsalzes mit Phosgen erhalten, siehe Arch. Pharm. 299, 174 (1966).

Verbindungen der Formel II und III, worin W und X für die Gruppe $—NR_1—CZ—OR$ steht, können aus den zugrundeliegenden Aminoverbindungen durch Reaktion mit einem Halogen(thio)ameisensäureester der Formel IV

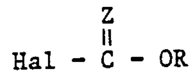

(IV)

oder einem (Thio)Kohlensäurediester der Formel V

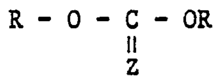

(V)

hergestellt werden ; hierbei steht Hal für ein Halogen, vorzugsweise Chlor oder Brom, Z steht für Sauerstoff oder Schwefel und R für einen aliphatischen oder aromatischen Rest, vorzugsweise für $C_1$-$C_4$-Alkyl, Phenyl oder Benzyl. Diese Reaktion wird bevorzugt in Gegenwart einer Base durchgeführt.

Die als Ausgangsverbindungen der Formel III benützten Amine sind bekannt oder können nach an sich bekannten Methoden zur Synthese heterocyclischer Amine hergestellt werden. Wir verweisen dazu auf folgende Literaturstellen : « The Chemistry of Heterocyclic Compounds », Band XVI Interscience Publishers, New York, London, worin sowohl Synthesen für 2-Aminopyrimidine wie auch für 2-Amino-1,3,5-triazine zu finden sind.

Diese Umsetzungen werden vorteilhafterweise in aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen —20° und +120 °C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator beschleunigt werden.

Die Wirkstoffe der Formel I sind stabile Verbindungen. Sie sind für Warmblüter wenig giftig.

Bei der Herstellung der Verbindungen der Formel I nach Gleichung [1] können in gewissen Fällen durch Addition einer Verbindung der Formel II an ein Ring-Stickstoffatom einer Verbindung der Formel III isomere Reaktionsprodukte der Formeln Ia bzw. Ib isoliert werden :

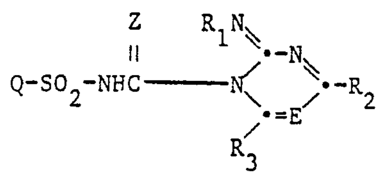

(Ia)

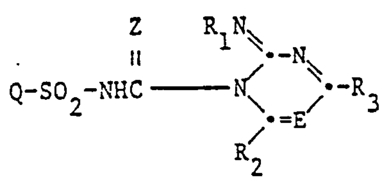

(Ib)

Hierbei haben $R_1$, $R_2$, $R_3$, Q, Z und E die unter Formel I angegebenen Bedeutungen. Auch diese isomeren Produkte Ia und Ib beeinflussen das Wachstum von Pflanzen. Die vorliegende Erfindung umfasst somit alle agrarchemisch verwendbaren Produkte und Produktgemische, die bei der Umsetzung der Verbindungen der Formel II mit III gebildet werden. Sulfonylharnstoffe mit Pyrimidin-, Pyridazin-, Pyrazin- oder Triazin-Ringen im Molekülteil Q sind neu.

Es wurde nun überraschend gefunden, dass die neuen Wirkstoffe der Formel I gute selektivherbizide Eigenschaften aufweisen und sich daher zur Verwendung in Kulturen von Nutzpflanzen eignen. Dabei kann teilweise auch beobachtet werden, dass selbst Problemunkräuter unterdrückt werden, denen bisher nur mit Totalherbiziden beizukommen war.

Einige Verbindungen der Formel I zeigen translozierende Eigenschaften, d. h. sie werden an der behandelten Stelle der Pflanze (Blatt, Stiel, Wurzel etc.) aufgenommen und an andere Stellen transportiert, wo sie ihre Wirkung entfalten. Sie folgen hierbei nicht nur der Richtung des Nährstofftransportes von den Wurzeln in die Blätter, sondern gelangen auch umgekehrt von den Blättern zu den Wurzeln. So gelingt es mit Hilfe dieser translozierenden Eigenschaften z. B. durch eine Oberflächenbehandlung

perenierende Unkräuter bis in die Wurzeln zu vernichten. Darüberhinaus wirken die Wirkstoffe der Formel I im Vergleich zu herkömmlichen Herbiziden bereits in sehr geringen Aufwandmengen.

Ferner können Verbindungen der Formel I die phytotoxischen Eigenschaften anderer herbizider Substanzen gegenüber gewissen Schadpflanzen verstärken und gegenüber einigen Kulturpflanzen vermindern. Dies kann zu einer Verringerung der gesamten Aufwandmenge an herbiziden Wirkstoffen und damit zu einer geringeren Umweltbelastung führen.

Werden die Aufwandmengen der erfindungsgemässen Wirkstoffe weiter gesenkt, so treten vor allem die wachstumsregulierenden Eigenschaften in den Vordergrund, dabei greifen die Verbindungen der Formel I gezielt in den Metabolismus der Pflanzen ein und führen insbesondere zu einer Reduktion des vegetativen Wachstums, oft zugunsten des generativen Wachstums. Dieser gezielte Eingriff in die physiologischen Vorgänge der Pflanzenentwicklung macht die Wirkstoffe der Formel I für verschiedene Zwecke verwendbar, insbesondere für solche, die mit der Arbeitseinsparung bei Massnahmen an Pflanzenkulturen oder der Ertragssteigerung im Zusammenhang stehen.

Hierin offenbarte Verbindungen der Formel I können als Herbizide und/oder als Wachstumsregulatoren eingesetzt werden. Nach bisherigen Erfahrungen gilt für die Applikation von Wachstumsregulatoren, dass die Aktivsubstanzen eine oder mehrere unterschiedliche Wirkungen bei den Pflanzen hervorrufen können. Diese verschiedenartigen Wirkungen hängen im wesentlichen vom Zeitpunkt der Anwendung, d. h. vom Entwicklungsstadium des Samens oder der Pflanze, der Art der Applikation sowie insbesondere von den angewendeten Konzentrationen ab. Derartige Effekte sind aber wiederum je nach Pflanzenart verschieden. Die Applikation von Verbindungen der Formel I eröffnet somit die Möglichkeit das Wachstum von Pflanzen in gewünschter Weise zu beeinflussen.

Mit den neuen Wirkstoffen der Formel I bzw. mit den neuen Mitteln wird das vegetative Pflanzenwachstum gedämpft. Durch diese Beeinflussung des Wachstums können die erfindungsgemäss verwendeten Wirkstoffe die Ernteausbeute von Pflanzen wesentlich erhöhen. So erfahren etwa Sojapflanzen und andere Leguminosen wie Bohnen, Erbsen oder Linsen eine Reduktion des vegetativen zugunsten des generativen Wachstums, wodurch eine unmittelbare Ertragssteigerung erzielt wird. Auch bei weiteren Pflanzenarten, z. B. bei Reben, Getreidesorten, Gräsern und Zierpflanzen wird das vegetative Wachstum in gewünschter Weise gehemmt. Daneben beobachtet man bei den behandelten Pflanzen eine deutliche Stärkung des Stützgewebes.

Eine im Vordergrund stehende Art der Pflanzenbeeinflussung beruht auf der besonderen Eigenschaft der Verbindungen der Formel I, bei bestimmten Pflanzen, insbesondere bei Getreidekulturen, eine gezielte Wuchshemmung hervorzurufen, die bei unveränderter Ertragsleistung die Knickfestigkeit der Pflanzen wesentlich erhöht. Daraus ergibt sich eine wirtschaftlich sehr interessante Methode des Schutzes von Pflanzenkulturen vor Lagerung durch Sturm oder Unwetter. Darüberhinaus gestattet eine gezielte Hemmung des vegetativen Wachstums bei vielen Kulturpflanzen eine dichtere Anpflanzung der Kulturen ; dies führt bei gleichem Fruchtansatz und gleicher Anbaufläche zu einer deutlichen Ertragssteigerung. Der Einsatz von Wuchshemmern bewirkt auch eine effektivere Nutzung der Nährstoffe, die nunmehr in verstärktem Masse der Blüten und Fruchtbildung zugute kommen. Auf diese Weise lassen sich höhere Ernteerträge bei gleichzeitig kleinerem Abfallanteil an vegetativen Pflanzenresten [z. B. Stroh, Kartoffelkraut, Rübenblätter] erzielen.

Hervorzuheben ist auch die Möglichkeit, mit den erfindungsgemässen Stoffen bzw. Mitteln bei verschiedenen Pflanzenarten, insbesondere bei Tabakpflanzen eine Hemmung des unerwünschten Geiztriebwuchses zu erreichen, wenn der Haupttrieb kurz vor der Blüte zum Zwecke der erstrebten Wuchsvergrösserung der Blätter abgeschnitten worden ist.

Die Erfindung betrifft somit eine herbizide und/oder wachstumsregulierende Verwendung der Verbindungen der Formel I. Eingeschlossen ist auch ein Verfahren zur pre- und post-emergen Unkrautbekämpfung, zur Hemmung des Pflanzenwuchses von monokotylen und dikotylen Pflanzen, insbesondere von Gräsern, Getreidesorten und Tabakgeiztrieben sowie zur Steigerung des Ernteertrages bei Leguminosen.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl-oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Aethylenoxid-Adduktes oder Phospholipide in Frage.

Als nichtionische Tenside kommen in erster Linie Polyglykoläther derivate von aliphatischen oder cycloaliphatischen Alkoholen, gesättigten oder ungesättigten Fettsäuren und Alkylphenolen in Frage, die 3 bis 30 Glykolöthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seinen Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u. a. in folgenden Publikationen beschrieben :

« Mc Cutcheon's Detergents and Emulsifiers Annual » MC Publishing Corp., Ridgewood, New Jersey, 1979. Sisley and Wood, « Encyclopedia of Surface Active Agents ». Chemical Publishing Co., Inc. New York, 1964.

Die pestiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen : ( % = Gewichtsprozent)

| | |
|---|---|
| Emulgierbare Konzentrate | |
| Aktiver Wirkstoff : | 1 bis 20 %, bevorzugt 5 bis 10 % |
| oberflächenaktives Mittel : | 5 bis 30 %, vorzugsweise 10 bis 20 % |
| flüssiges Trägermittel : | 50 bis 94 %, vorzugsweise 70 bis 85 % |
| Stäube : | |
| Aktiver Wirkstoff : | 0,1 bis 10 %, vorzugsweise 0,1 bis 1 % |
| festes Trägermittel : | 99,9 bis 90 %, vorzugsweise 99,9 bis 99 % |
| Suspension-Konzentrate | |
| Aktiver Wirkstoff : | 5 bis 75 %, vorzugsweise 10 bis 50 % |
| Wasser : | 94 bis 25 %, vorzugsweise 90 bis 30 % |
| oberflächenaktives Mittel : | 1 bis 40 %, vorzugsweise 2 bis 30 % |
| Benetzbare Pulver | |
| Aktiver Wirkstoff : | 0,5 bis 90 %, vorzugsweise 1 bis 80 % |
| oberflächenaktives Mittel : | 0,5 bis 20 %, vorzugsweise 1 bis 15 % |
| festes Trägermaterial : | 5 bis 95 %, vorzugsweise 15 bis 90 % |
| Granulate | |
| Aktiver Wirkstoff : | 0,5 bis 30 %, vorzugsweise 3 bis 15 % |
| festes Trägermittel : | 99,5 bis 70 %, vorzugsweise 97 bis 85 % |

Während als Handelsware eher konzentrierte Mittel bevorzugt werden, verwendet der Endverbraucher in der Regel verdünnte Mitte. Die Anwendungsformen können bis hinab zu 0,001 % an Wirkstoff verdünnt werden. Die Aufwandmengen betragen in der Regel 0,01 bis 10 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die Erfindung umfasst auch derartige landwirtschaftlich verwendbare Mittel, die als mindestens eine Aktivsubstanz eine Verbindung der Formel I enthalten.

Darüberhinaus schliesst die vorliegende Erfindung auch die Herstellung solcher Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

In den nachfolgenden Beispielen sind Temperaturen in Celsiusgraden angegeben, Prozentangaben und Teile beziehen sich auf das Gewicht.

## Herstellungsbeispiel

### Beispiel 1

a) 3-Chlorpyridazin-6-sulfonamid

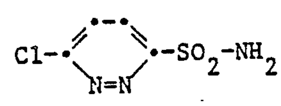

Eine Suspension von 20 g 3-Chlorpyridazin-6-thiol und 54,4 g Kaliumhydrogenfluorid in 200 ml 50 proz. Essigsäure wurde auf 0 °C abgekühlt. Unter kräftigem Rühren und bei einer Temperatur zwischen 0-5 °C wurde während 3 Stunden Chlor in die Suspension eingeleitet. Danach wurde das Reaktionsgemisch mit 300 ml kaltem Wasser versetzt, das ausgefallene Sulfonylchlorid abfiltriert und mit kaltem Wasser gewaschen. Inzwhischen wurde in einem Sulfierkolben ein Ueberschuss von flüssigem Ammoniak vorgelegt. Unter Rühren wurde dann portionsweise das Sulfonylchlorid zugegeben. Das Reaktionsgemisch wurde für einige Stunden weitergerührt, mit 100 ml kaltem Wasser verdünnt, von unlöslichen Teilen abfiltriert und das Filtrat mit verdünnter Salzsäure auf pH 4 gestellt. Nach dem Abfiltrieren erhält man 11g von der Substanz der obigen Formel mit dem Smp. : 150-152° (Zersetzung)

b) N-(3-Chlorpyridazin-6-sulfonyl)-phenylcarbamat

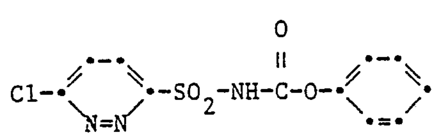

Zu einer Lösung von 10,6 g 3-Chlorpyridazin-6-sulfonamid in 50 ml absolutem Dimethylformamid werden unter Stickstoffatmosphäre bei Raumtemperatur 2,5 g einer 55 %igen Natriumhydriddispersion in Oel portionsweise während 15 Minuten zugegeben und weitere 15 Minuten bei 35° weiter reagieren lassen. Nach dem Abkühlen auf 25° wurde diese Lösung mit 12,4 g Diphenylcarbonat versetzt. Das Reaktionsgemisch wird 2 Stunden bei Raumtemperatur ausgerührt, auf ein Gemisch von 500 ml Eiswasser und 50 ml Salzsäure gegossen. Der ausgefallene Niederschlag wird abgenutscht, mit kaltem Wasser gewaschen und im Vakuumschrank getrocknet.

Man erhält 14,4 g von der Substanz der obigen Formel mit dem Smp. : 136°.

c) N-(3-Chlorpyridazin-6-sulfonyl)-N'-(4-methoxy-6-methyl-1.3.5-triazin-2-yl)-harnstoff

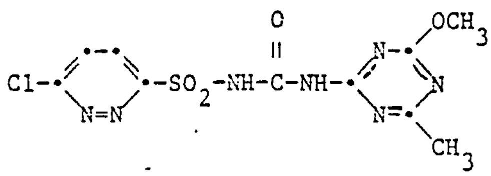

Ein Gemisch von 14,2 g N-(3-Chlorpyridazin-6-sulfonyl)-phenylcarbamat und 6,3 g 2-Amino-4-methoxy-6-methyl-1.3.5-triazin in 120 ml absolutem Dioxan wird 1 Stunde lang am Rückfluss gekocht. Nach Beendigung der Reaktion wurde unter Vakuum eingedampft, der Rückstand in einer gesättigten wässrigen Natriumbicarbonatlösung aufgenommen und die unlöslichen Teile mit Methylenchlorid extrahiert. Die wässrige Phase wurde mit verdünnter Salzsäure angesäuert und erneut mit Methylenchlorid extrahiert.

Nach dem Trocknen der Methylenchloridlösung über Natriumsulfat und Eindampfen erhält man 7 g von der Substanz der obigen Formel mit dem Smp. : 135°.

(Siehe Tabellen Seite 10 ff.)

Auf analoge Weise können die in den folgenden Tabellen aufgelisteten Zwischen- und Endprodukte hergestellt werden.

Tabelle 1 $Q - SO_2 - NH_2$

| Verb.Nr. | Q | Abkürzung | phys. Daten |
|---|---|---|---|
| 1.1 | 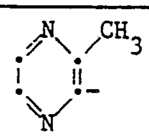 | Q 1 | Smp. 109-110°C |
| 1.2 | 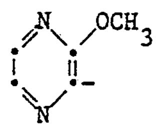 | Q 2 | Smp. 115-120°C |
| 1.3 | 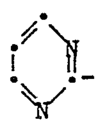 | Q 3 | |
| 1.4 | 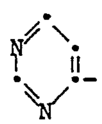 | Q 4 | |
| 1.5 | 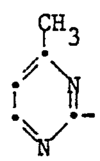 | Q 5 | |
| 1.6 | 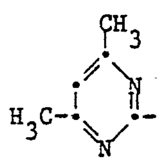 | Q 6 | |
| 1.7 | 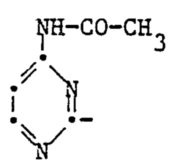 | Q 7 | |
| 1.8 | 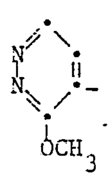 | Q 8 | |

Tabelle 1 Q = $SO_2 - NH_2$ (Fortsetzung)

| Verb.Nr. | Q | Abkürzung | phys. Daten |
|---|---|---|---|
| 1.9 | 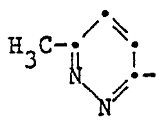 | Q 9 | |
| 1.10 | 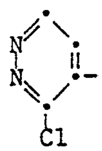 | Q 10 | |
| 1.11 | 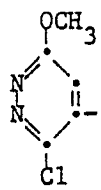 | Q 11 | |
| 1.12 | 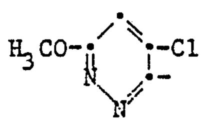 | Q 12 | |
| 1.13 | 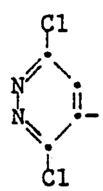 | Q 13 | |
| 1.14 | 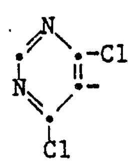 | Q 14 | |
| 1.15 | 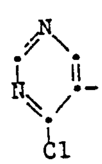 | Q 15 | |

Tabelle 1 $Q - SO_2 - NH_2$ (Fortsetzung)

| Verb.Nr. | Q | Abkürzung | phys. Daten |
|---|---|---|---|
| 1.16 | 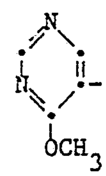 | Q 16 | |
| 1.17 | 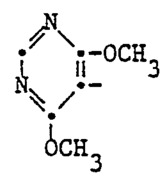 | Q 17 | |
| 1.18 | 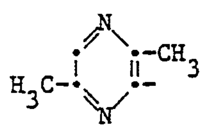 | Q 18 | |
| 1.19 | 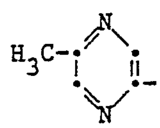 | Q 19 | |
| 1.20 | 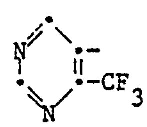 | Q 20 | |
| 1.21 | 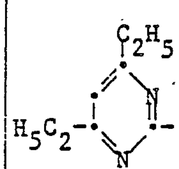 | Q 21 | |
| 1.22 | 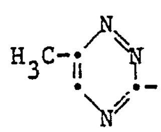 | Q 22 | |
| 1.23 | 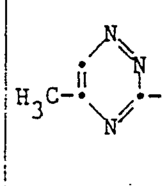 | Q 23 | |

Tabelle 1 $Q - SO_2 - NH_2$ (Fortsetzung)

| Verb.Nr. | Q | Abkürzung | phys. Daten |
|---|---|---|---|
| 1.24 | 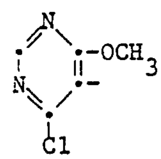 | Q 24 | |
| 1.25 | 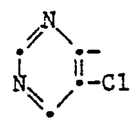 | Q 25 | |
| 1.26 | 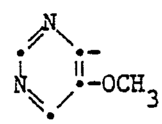 | Q 26 | |
| 1.27 | 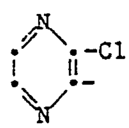 | Q 27 | |
| 1.28 | 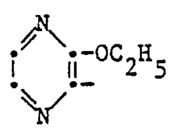 | Q 28 | |
| 1.29 | 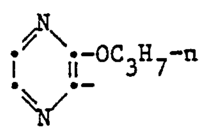 | Q 29 | |
| 1.30 | 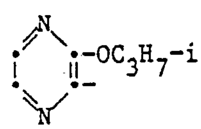 | Q 30 | |
| 1.31 | 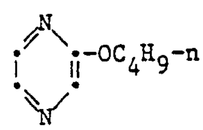 | Q 31 | |
| 1.32 | 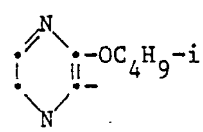 | Q 32 | |

Tabelle 1 $Q - SO_2 - NH_2$ (Fortsetzung)

| Verb. Nr. | Q | Abkürzung | phys. Daten |
|---|---|---|---|
| 1.33 | 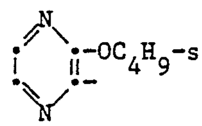 | Q 33 | |
| 1.34 | 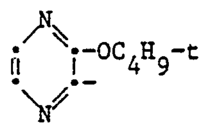 | Q 34 | |
| 1.35 | 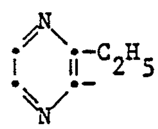 | Q 35 | |
| 1.36 | 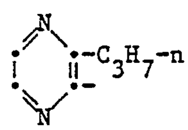 | Q 36 | |
| 1.37 | 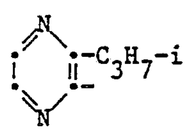 | Q 37 | |
| 1.38 | 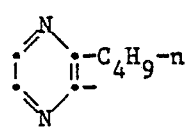 | Q 38 | |
| 1.39 | 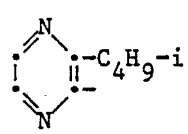 | Q 39 | |

Tabelle 2 $Q - SO_2 - W$

| Verb.Nr. | Q | W | phys. Daten |
|---|---|---|---|
| 2.1 | Q 1 | $-NH-CO-OCH_3$ | |
| 2.2 | Q 2 | $-NH-CO-OCH_3$ | |
| 2.3 | Q 3 | $-NH-CO-OCH_3$ | |
| 2.4 | Q 4 | $-NH-CO-OCH_3$ | |
| 2.5 | Q 5 | $-NH-CO-OCH_3$ | |
| 2.6 | Q 6 | $-NH-CO-OCH_3$ | |
| 2.7 | Q 7 | $-NH-CO-OCH_3$ | |
| 2.8 | Q 8 | $-NH-CO-OCH_3$ | |
| 2.9 | Q 9 | $-NH-CO-OCH_3$ | |
| 2.10 | Q 10 | $-NH-CO-OCH_3$ | |
| 2.11 | Q 11 | $-NH-CO-OCH_3$ | |
| 2.12 | Q 12 | $-NH-CO-OCH_3$ | |
| 2.13 | Q 13 | $-NH-CO-OCH_3$ | |
| 2.14 | Q 14 | $-NH-CO-OCH_3$ | |
| 2.15 | Q 15 | $-NH-CO-OCH_3$ | |
| 2.16 | Q 16 | $-NH-CO-OCH_3$ | |
| 2.17 | Q 17 | $-NH-CO-OCH_3$ | |
| 2.18 | Q 18 | $-NH-CO-OCH_3$ | |
| 2.19 | Q 19 | $-NH-CO-OCH_3$ | |
| 2.20 | Q 20 | $-NH-CO-OCH_3$ | |
| 2.21 | Q 21 | $-NH-CO-OCH_3$ | |
| 2.22 | Q 22 | $-NH-CO-OCH_3$ | |
| 2.23 | Q 23 | $-NH-CO-OCH_3$ | |
| 2.24 | Q 24 | $-NH-CO-OCH_3$ | |
| 2.25 | Q 25 | $-NH-CO-OCH_3$ | |
| 2.26 | Q 26 | $-NH-CO-OCH_3$ | |
| 2.27 | Q 27 | $-NH-CO-OCH_3$ | |
| 2.28 | Q 1 | $-NH-CO-O-C_6H_5$ | Smp. 141-143°C |
| 2.29 | Q 2 | $-NH-CO-O-C_6H_5$ | Smp. 135-136°C |

Tabelle 2 Q - $SO_2$ - W (Fortsetzung)

| Verb.Nr. | Q | W | phys. Daten |
|---|---|---|---|
| 2.30 | Q 3 | $-NH-CO-O-C_6H_5$ | |
| 2.31 | Q 4 | $-NH-CO-O-C_6H_5$ | |
| 2.32 | Q 5 | $-NH-CO-O-C_6H_5$ | |
| 2.33 | Q 6 | $-NH-CO-O-C_6H_5$ | |
| 2.34 | Q 7 | $-NH-CO-O-C_6H_5$ | |
| 2.35 | Q 8 | $-NH-CO-O-C_6H_5$ | |
| 2.36 | Q 9 | $-NH-CO-O-C_6H_5$ | |
| 2.37 | Q 10 | $-NH-CO-O-C_6H_5$ | |
| 2.38 | Q 11 | $-NH-CO-O-C_6H_5$ | |
| 2.39 | Q 12 | $-NH-CO-O-C_6H_5$ | |
| 2.40 | Q 13 | $-NH-CO-O-C_6H_5$ | |
| 2.41 | Q 14 | $-NH-CO-O-C_6H_5$ | |
| 2.42 | Q 15 | $-NH-CO-O-C_6H_5$ | |
| 2.43 | Q 16 | $-NH-CO-O-C_6H_5$ | |
| 2.44 | Q 17 | $-NH-CO-O-C_6H_5$ | |
| 2.45 | Q 18 | $-NH-CO-O-C_6H_5$ | |
| 2.46 | Q 19 | $-NH-CO-O-C_6H_5$ | |
| 2.47 | Q 20 | $-NH-CO-O-C_6H_5$ | |
| 2.48 | Q 21 | $-NH-CO-O-C_6H_5$ | |
| 2.49 | Q 22 | $-NH-CO-O-C_6H_5$ | |
| 2.50 | Q 23 | $-NH-CO-O-C_6H_5$ | |
| 2.51 | Q 24 | $-NH-CO-O-C_6H_5$ | |
| 2.52 | Q 25 | $-NH-CO-O-C_6H_5$ | |
| 2.53 | Q 26 | $-NH-CO-O-C_6H_5$ | |
| 2.54 | Q 27 | $-NH-CO-O-C_6H_5$ | |
| 2.55 | Q 1 | $-NH-CO-OC_2H_5$ | |
| 2.56 | Q 2 | $-NH-CO-OC_2H_5$ | |
| 2.57 | Q 3 | $-NH-CO-OC_2H_5$ | |
| 2.58 | Q 4 | $-NH-CO-OC_2H_5$ | |

Tabelle 2 Q - $SO_2$ - W (Fortsetzung)

| Verb.Nr. | Q | W | phys. Daten |
|---|---|---|---|
| 2.59 | Q 5 | $-NH-CO-OC_2H_5$ | |
| 2.60 | Q 6 | $-NH-CO-OC_2H_5$ | |
| 2.61 | Q 7 | $-NH-CO-OC_2H_5$ | |
| 2.62 | Q 8 | $-NH-CO-OC_2H_5$ | |
| 2.63 | Q 9 | $-NH-CO-OC_2H_5$ | |
| 2.64 | Q 10 | $-NH-CO-OC_2H_5$ | |
| 2.65 | Q 11 | $-NH-CO-OC_2H_5$ | |
| 2.66 | Q 12 | $-NH-CO-OC_2H_5$ | |
| 2.67 | Q 13 | $-NH-CO-OC_2H_5$ | |
| 2.68 | Q 14 | $-NH-CO-OC_2H_5$ | |
| 2.69 | Q 15 | $-NH-CO-OC_2H_5$ | |
| 2.70 | Q 16 | $-NH-CO-OC_2H_5$ | |
| 2.71 | Q 17 | $-NH-CO-OC_2H_5$ | |
| 2.72 | Q 18 | $-NH-CO-OC_2H_5$ | |
| 2.73 | Q 19 | $-NH-CO-OC_2H_5$ | |
| 2.74 | Q 20 | $-NH-CO-OC_2H_5$ | |
| 2.75 | Q 21 | $-NH-CO-OC_2H_5$ | |
| 2.76 | Q 22 | $-NH-CO-OC_2H_5$ | |
| 2.77 | Q 23 | $-NH-CO-OC_2H_5$ | |
| 2.78 | Q 24 | $-NH-CO-OC_2H_5$ | |
| 2.79 | Q 25 | $-NH-CO-OC_2H_5$ | |
| 2.80 | Q 26 | $-NH-CO-OC_2H_5$ | |
| 2.81 | Q 27 | $-NH-CO-OC_2H_5$ | |
| 2.82 | Q 28 | $-NH-CO-OC_2H_5$ | |
| 2.83 | Q 1 | -N=C=O | |
| 2.84 | Q 2 | -N=C=O | |
| 2.85 | Q 3 | -N=C=O | |
| 2.86 | Q 4 | -N=C=O | |
| 2.87 | Q 5 | -N=C=O | |

Tabelle 2 $Q - SO_2 - W$ (Fortsetzung)

| Verb.Nr. | Q | W | phys. Daten |
|---|---|---|---|
| 2.88 | Q 6 | -N=C=O | |
| 2.89 | Q 8 | -N=C=O | |
| 2.90 | Q 9 | -N=C=O | |
| 2.91 | Q 10 | -N=C=O | |
| 2.92 | Q 11 | -N=C=O | |
| 2.93 | Q 12 | -N=C=O | |
| 2.94 | Q 13 | -N=C=O | |
| 2.95 | Q 14 | -N=C=O | |
| 2.96 | Q 15 | -N=C=O | |
| 2.97 | Q 16 | -N=C=O | |
| 2.98 | Q 17 | -N=C=O | |
| 2.99 | Q 18 | -N=C=O | |
| 2.100 | Q 19 | -N=C=O | |
| 2.101 | Q 20 | -N=C=O | |
| 2.102 | Q 21 | -N=C=O | |
| 2.103 | Q 22 | -N=C=O | |
| 2.104 | Q 23 | -N=C=O | |
| 2.105 | Q 24 | -N=C=O | |
| 2.106 | Q 25 | -N=C=O | |
| 2.107 | Q 26 | -N=C=O | |
| 2.108 | Q 27 | -N=C=O | |

Tabelle 3 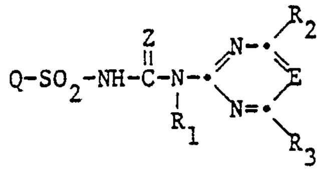

| Verb. Nr. | Q | Z | E | $R_1$ | $R_2$ | $R_3$ | Smp. [°C] |
|---|---|---|---|---|---|---|---|
| 3.1 | Q 3 | O | N | H | $CH_3$ | $OCH_3$ | |
| 3.2 | Q 3 | O | CH | H | $CH_3$ | $OCH_3$ | |
| 3.3 | Q 4 | O | N | H | $OCH_3$ | $OCH_3$ | |
| 3.4 | Q 5 | S | N | $CH_3$ | $CH_3$ | $CH_3$ | |
| 3.5 | Q 7 | O | N | H | $CH_3$ | $OCH_3$ | |
| 3.6 | Q 6 | O | N | H | $CH_3$ | $OCH_3$ | |
| 3.7 | Q 8 | O | N | H | $CH_3$ | $OCH_3$ | |
| 3.8 | Q 8 | O | CH | H | $CH_3$ | $OCH_3$ | |
| 3.9 | Q 9 | O | N | $CH_3$ | $CH_3$ | $OCH_3$ | |
| 3.10 | Q 10 | O | N | H | $CH_3$ | $OCH_3$ | |
| 3.11 | Q 10 | O | CH | H | $CH_3$ | $OCH_3$ | |
| 3.12 | Q 11 | O | N | H | $OCH_3$ | $OCH_3$ | |
| 3.13 | Q 11 | O | CH | H | $CH_3$ | $OCH_3$ | |
| 3.14 | Q 27 | O | N | H | $CH_3$ | $OCH_3$ | |
| 3.15 | Q 27 | O | CH | H | $CH_3$ | $OCH_3$ | |
| 3.16 | Q 13 | S | N | H | $CH_3$ | $CH_2$ | |
| 3.17 | Q 13 | O | CH | H | H | H | |
| 3.18 | Q 18 | O | N | H | $OCH_3$ | $OCH_3$ | |
| 3.19 | Q 19 | O | N | $CH_3$ | $CH_3$ | $CH_3$ | |
| 3.20 | Q 18 | O | N | H | $CH_3$ | $OCH_3$ | |
| 3.21 | Q 3 | O | CH | H | $CH_3$ | $CH_3$ | 160-161 |
| 3.22 | Q 3 | O | N | H | $CH_3$ | $OCH_3$ | |
| 3.23 | Q 5 | O | CH | H | $CH_3$ | $OCH_3$ | |
| 3.24 | Q 5 | O | N | H | $CH_3$ | $OCH_3$ | |
| 3.25 | Q 6 | O | CH | H | $CH_3$ | $OCH_3$ | |
| 3.26 | Q 17 | O | N | H | $CH_3$ | $OCH_3$ | |
| 3.27 | Q 17 | O | N | H | $OCH_3$ | $OCH_3$ | |
| 3.28 | Q 6 | O | N | H | $CH_3$ | $OCH_3$ | |

Tabelle 3 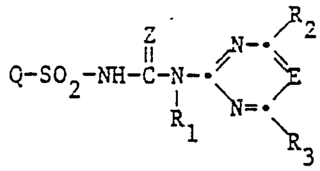 (Fortsetzung)

| Verb. Nr. | Q | Z | E | $R_1$ | $R_2$ | $R_3$ | |
|---|---|---|---|---|---|---|---|
| 3.29 | Q 20 | O | N | $CH_3$ | $CH_3$ | $CH_3$ | |
| 3.30 | Q 7 | S | N | H | $CH_3$ | $OC_3H_7$-n | |
| 3.31 | Q 21 | O | N | H | $CH_3$ | $OC_2H_5$ | |
| 3.32 | Q 13 | O | N | H | $CH_3$ | $OCH_3$ | |
| 3.33 | Q 13 | O | CH | H | $CH_3$ | $OCH_3$ | |
| 3.34 | Q 22 | S | CH | $CH_3$ | $CH_3$ | $CH_3$ | |
| 3.35 | Q 23 | O | N | H | $OCH_3$ | $OCH_3$ | |
| 3.36 | Q 1 | O | N | H | $CH_3$ | $OCH_3$ | 153-154 |
| 3.37 | Q 1 | O | N | H | $OCH_3$ | $N(CH_3)_2$ | 154-155 |
| 3.38 | Q 1 | O | N | H | $OCH_3$ | $OC_2H_5$ | |
| 3.39 | Q 1 | O | N | H | $OCH_3$ | $OCH(CH_3)_2$ | |
| 3.40 | Q 1 | O | N | H | $OCH_3$ | $OCH_2CF_3$ | |
| 3.41 | Q 1 | O | N | H | $C_2H_5$ | $OCH_3$ | 139-141 |
| 3.42 | Q 1 | O | N | H | $OC_2H_5$ | $OC_2H_5$ | |
| 3.43 | Q 1 | O | N | H | $SCH_3$ | $OCH_3$ | |
| 3.44 | Q 1 | O | N | H | $CH_3$ | $OC_2H_5$ | 143-144 |
| 3.45 | Q 1 | O | CH | H | $CH_3$ | $CH_3$ | 165-167 |
| 3.46 | Q 1 | O | CH | H | $CH_3$ | $OCH_3$ | 138-141 |
| 3.47 | Q 1 | O | CH | H | $CH_3$ | $OCHF_2$ | 142-145 |
| 3.48 | Q 1 | O | CH | H | $CH_3$ | $N(CH_3)_2$ | |
| 3.49 | Q 1 | O | CH | H | $OCH_3$ | $OCH_3$ | 160-163 |
| 3.50 | Q 1 | O | CH | H | $OCHF_2$ | $OCHF_2$ | |
| 3.51 | Q 1 | O | CH | H | $OCH_3$ | $OCHF_2$ | |
| 3.52 | Q 1 | O | CH | H | $OCH_3$ | Cl | |
| 3.53 | Q 1 | O | CH | H | $OCH_3$ | F | |
| 3.54 | Q 1 | O | CH | H | $OCH_3$ | $N(CH_3)_2$ | |
| 3.55 | Q 1 | O | CH | H | $CH_2F$ | $OCH_3$ | |

Tabelle 3 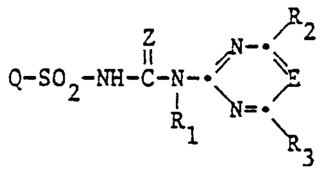 (Fortsetzung)

| Verb. Nr. | Q | Z | E | $R_1$ | $R_2$ | $R_3$ | |
|---|---|---|---|---|---|---|---|
| 3.56 | Q 1 | O | CH | H | $OCHF_2$ | $N(CH_3)_2$ | |
| 3.57 | Q 1 | O | CH | H | $OCH_3$ | $OCF_2$-$CHF_2$ | |
| 3.58 | Q 1 | O | CH | H | $CH_3$ | $OCF_2$-$CHF_2$ | |
| 3.59 | Q 2 | O | N | H | $CH_3$ | $OCH_3$ | 135-137 |
| 3.60 | Q 2 | O | N | H | $OCH_3$ | $N(CH_3)_2$ | 180-181 |
| 3.61 | Q 2 | O | N | H | $OCH_3$ | $OC_2H_5$ | |
| 3.62 | Q 2 | O | N | H | $OCH_3$ | $OCH(CH_3)_2$ | |
| 3.63 | Q 2 | O | N | H | $OCH_3$ | $OCH_2CF_3$ | |
| 3.64 | Q 2 | O | N | H | $C_2H_5$ | $OCH_3$ | 122-124 |
| 3.65 | Q 2 | O | N | H | $OC_2H_5$ | $OC_2H_5$ | |
| 3.66 | Q 2 | O | N | H | $SCH_3$ | $OCH_3$ | |
| 3.67 | Q 2 | O | N | H | $CH_3$ | $OC_2H_5$ | 123-125 |
| 3.68 | Q 2 | O | CH | H | $CH_3$ | $CH_3$ | 166-167 |
| 3.69 | Q 2 | O | CH | H | $CH_3$ | $OCH_3$ | 139-141 |
| 3.70 | Q 2 | O | CH | H | $CH_3$ | $OCHF_2$ | 116-123 |
| 3.71 | Q 2 | O | CH | H | $CH_3$ | $N(CH_3)_2$ | |
| 3.72 | Q 2 | O | CH | H | $OCH_3$ | $OCH_3$ | 143-147 |
| 3.73 | Q 2 | O | CH | H | $OCHF_2$ | $OCHF_2$ | |
| 3.74 | Q 2 | O | CH | H | $OCH_3$ | $OCHF_2$ | |
| 3.75 | Q 2 | O | CH | H | $OCH_3$ | Cl | |
| 3.76 | Q 2 | O | CH | H | $OCH_3$ | F | |
| 3.77 | Q 2 | O | CH | H | $OCH_3$ | $N(CH_3)_2$ | |
| 3.78 | Q 2 | O | CH | H | $CH_2F$ | $OCH_3$ | |
| 3.79 | Q 2 | O | CH | H | $OCHF_2$ | $N(CH_3)_2$ | |
| 3.80 | Q 2 | O | CH | H | $OCH_3$ | $OCF_2$-$CHF_2$ | |
| 3.81 | Q 2 | O | CH | H | $CH_3$ | $OCF_2$-$CHF_2$ | |
| 3.82 | Q 14 | O | N | H | $CH_3$ | $OCH_3$ | |

Tabelle 3 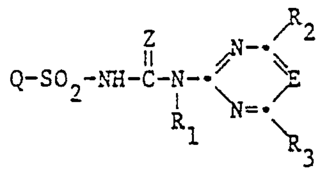 (Fortsetzung)

| Verb. Nr. | Q | Z | E | $R_1$ | $R_2$ | $R_3$ | Smp. [°C] |
|---|---|---|---|---|---|---|---|
| 3.83 | Q 14 | O | CH | H | $CH_3$ | $OCH_3$ | |
| 3.84 | Q 14 | O | CH | H | $CH_3$ | $CH_3$ | |
| 3.85 | Q 14 | O | CH | H | $OCH_3$ | $OCH_3$ | |
| 3.86 | Q 14 | O | CH | H | $CH_3$ | $OCHF_2$ | |
| 3.87 | Q 24 | O | N | H | $CH_3$ | $OCH_3$ | |
| 3.88 | Q 24 | O | CH | H | $CH_3$ | $OCH_3$ | |
| 3.89 | Q 24 | O | CH | H | $CH_3$ | $OCHF_2$ | |
| 3.90 | Q 25 | O | N | H | $CH_3$ | $OCH_3$ | |
| 3.91 | Q 25 | O | CH | H | $CH_3$ | $OCH_3$ | |
| 3.92 | Q 26 | O | N | H | $CH_3$ | $OCH_3$ | |
| 3.93 | Q 26 | O | CH | H | $CH_3$ | $OCH_3$ | |
| 3.94 | Q 15 | O | CH | H | $CH_3$ | $OCH_3$ | |
| 3.95 | Q 15 | O | N | H | $CH_3$ | $OCH_3$ | |
| 3.96 | Q 15 | O | CH | H | $CH_3$ | $OCHF_2$ | |
| 3.97 | Q 16 | O | N | H | $CH_3$ | $OCH_3$ | |
| 3.98 | Q 16 | O | CH | H | $CH_3$ | $OCH_3$ | |
| 3.99 | Q 17 | O | N | H | $CH_3$ | $OCH_3$ | |
| 3.100 | Q 17 | O | CH | H | $CH_3$ | $OCH_3$ | |
| 3.101 | Q 27 | O | N | H | $OCH_3$ | $OC_2H_5$ | |
| 3.102 | Q 27 | O | N | H | $OCH_3$ | $OCH(CH_3)_2$ | |
| 3.103 | Q 27 | O | N | H | $CH_3$ | $OCH_3$ | |
| 3.104 | Q 27 | O | N | H | $OCH_3$ | $N(CH_3)_2$ | |
| 3.105 | Q 27 | O | N | H | $OCH_3$ | $OCH_2CF_3$ | |
| 3.106 | Q 27 | O | N | H | $C_2H_5$ | $OCH_3$ | |
| 3.107 | Q 27 | O | N | H | $OCH_3$ | $OCH_3$ | |
| 3.108 | Q 27 | O | N | H | $CH_3$ | $OC_2H_5$ | |
| 3.109 | Q 27 | O | CH | H | $CH_3$ | $CH_3$ | |
| 3.110 | Q 27 | O | CH | H | $CH_3$ | $OCH_3$ | |

Tabelle 3 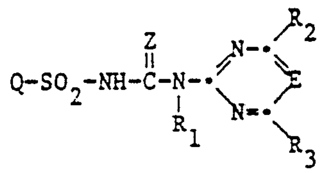 (Fortsetzung)

| Verb. Nr. | Q | Z | E | $R_1$ | $R_2$ | $R_3$ | Smp. [°C] |
|---|---|---|---|---|---|---|---|
| 3.111 | Q 27 | O | CH | $CH_3$ | $CH_3$ | $OC_2H_5$ | |
| 3.112 | Q 27 | O | CH | H | $OCH_3$ | $OCH_3$ | |
| 3.113 | Q 27 | O | CH | H | $OCHF_2$ | $OCHF_2$ | |
| 3.114 | Q 27 | O | CH | H | $OCH_3$ | $OCHF_2$ | |
| 3.115 | Q 30 | O | N | H | $OCH_3$ | $OC_2H_5$ | |
| 3.116 | Q 30 | O | N | H | $OCH_3$ | $OCH(CH_3)_2$ | |
| 3.117 | Q 30 | O | N | H | $CH_3$ | $OCH_3$ | |
| 3.118 | Q 30 | O | N | H | $OCH_3$ | $N(CH_3)_2$ | |
| 3.119 | Q 30 | O | N | H | $OCH_3$ | $OCH_2CF_3$ | |
| 3.120 | Q 30 | O | N | H | $C_2H_5$ | $OCH_3$ | |
| 3.121 | Q 30 | O | N | H | $OCH_3$ | $OCH_3$ | |
| 3.122 | Q 30 | O | N | H | $CH_3$ | $OC_2H_5$ | |
| 3.123 | Q 30 | O | CH | H | $CH_3$ | $CH_3$ | |
| 3.124 | Q 30 | O | CH | H | $CH_3$ | $OCH_3$ | |
| 3.125 | Q 30 | O | CH | H | $CH_3$ | $OCHF_2$ | |
| 3.126 | Q 30 | O | CH | H | $OCH_3$ | $OCH_3$ | |
| 3.127 | Q 30 | O | CH | H | $OCHF_2$ | $OCHF_2$ | |
| 3.128 | Q 30 | O | CH | H | $OCH_3$ | $OCHF_2$ | |
| 3.129 | Q 32 | O | N | H | $OCH_3$ | $OC_2H_5$ | |
| 3.130 | Q 32 | O | N | H | $OCH_3$ | $OCH(CH_3)_2$ | |
| 3.131 | Q 32 | O | N | H | $CH_3$ | $OCH_3$ | |
| 3.132 | Q 32 | O | N | H | $OCH_3$ | $N(CH_3)_2$ | |
| 3.133 | Q 32 | O | N | H | $OCH_3$ | $OCH_2CF_3$ | |
| 3.134 | Q 32 | O | N | H | $C_2H_5$ | $OCH_3$ | |
| 3.135 | Q 32 | O | N | H | $OCH_3$ | $OCH_3$ | |
| 3.136 | Q 32 | O | N | H | $CH_3$ | $OC_2H_5$ | |
| 3.137 | Q 32 | O | CH | H | $CH_3$ | $CH_3$ | |
| 3.138 | Q 32 | O | CH | H | $CH_3$ | $OCH_3$ | |

Tabelle 3 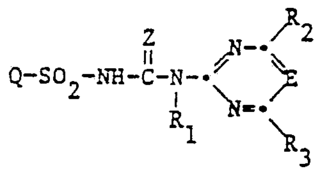 (Fortsetzung)

| Verb. Nr. | Q | Z | E | $R_1$ | $R_2$ | $R_3$ | Smp [°C] |
|---|---|---|---|---|---|---|---|
| 3.139 | Q 32 | 0 | CH | H | $CH_3$ | $OCHF_2$ | |
| 3.140 | Q 32 | 0 | CH | H | $OCH_3$ | $OCH_3$ | |
| 3.141 | Q 32 | 0 | CH | H | $OCHF_2$ | $OCHF_2$ | |
| 3.142 | Q 32 | 0 | CH | H | $OCH_3$ | $OCHF_2$ | |
| 3.143 | Q 33 | 0 | N | H | $OCH_3$ | $OC_2H_5$ | |
| 3.144 | Q 33 | 0 | N | H | $OCH_3$ | $OCH(CH_3)_2$ | |
| 3.145 | Q 33 | 0 | N | H | $CH_3$ | $OCH_3$ | |
| 3.146 | Q 33 | 0 | N | H | $OCH_3$ | $N(CH_3)_2$ | |
| 3.147 | Q 33 | 0 | N | H | $OCH_3$ | $OCH_2CF_3$ | |
| 3.148 | Q 33 | 0 | N | H | $C_2H_5$ | $OCH_3$ | |
| 3.149 | Q 33 | 0 | N | H | $OCH_3$ | $OCH_3$ | |
| 3.150 | Q 33 | 0 | N | H | $CH_3$ | $OC_2H_5$ | |
| 3.151 | Q 33 | 0 | CH | H | $CH_3$ | $CH_3$ | |
| 3.152 | Q 33 | 0 | CH | H | $CH_3$ | $OCH_3$ | |
| 3.153 | Q 33 | 0 | CH | H | $CH_3$ | $OCHF_2$ | |
| 3.154 | Q 33 | 0 | CH | H | $OCH_3$ | $OCH_3$ | |
| 3.155 | Q 33 | 0 | CH | H | $OCHF_2$ | $OCHF_2$ | |
| 3.156 | Q 33 | 0 | CH | H | $OCH_3$ | $OCHF_2$ | |
| 3.157 | Q 34 | 0 | N | H | $OCH_3$ | $OC_2H_5$ | |
| 3.158 | Q 34 | 0 | N | H | $OCH_3$ | $OCH(CH_3)_2$ | |
| 3.159 | Q 34 | 0 | N | H | $CH_3$ | $OCH_3$ | |
| 3.160 | Q 34 | 0 | N | H | $OCH_3$ | $N(CH_3)_2$ | |
| 3.161 | Q 34 | 0 | N | H | $OCH_3$ | $OCH_2CF_3$ | |
| 3.162 | Q 34 | 0 | N | H | $C_2H_5$ | $OCH_3$ | |
| 3.163 | Q 34 | 0 | N | H | $OCH_3$ | $OCH_3$ | |
| 3.164 | Q 34 | 0 | N | H | $CH_3$ | $OC_2H_5$ | |
| 3.165 | Q 34 | 0 | CH | H | $CH_3$ | $CH_3$ | |
| 3.166 | Q 34 | 0 | CH | H | $CH_3$ | $OCH_3$ | |

Tabelle 3 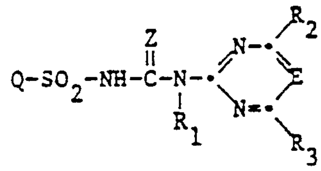 (Fortsetzung)

| Verb. Nr. | Q | Z | E | $R_1$ | $R_2$ | $R_3$ | Smp. [°C] |
|---|---|---|---|---|---|---|---|
| 3.167 | Q 34 | O | CH | H | $CH_3$ | $OCHF_2$ | |
| 3.168 | Q 34 | O | CH | H | $OCH_3$ | $OCH_3$ | |
| 3.169 | Q 34 | O | CH | H | $OCHF_2$ | $OCHF_2$ | |
| 3.170 | Q 34 | O | CH | H | $OCH_3$ | $OCHF_2$ | |
| 3.171 | Q 37 | O | N | H | $OCH_3$ | $OC_2H_5$ | |
| 3.172 | Q 37 | O | N | H | $OCH_3$ | $OCH(CH_3)_2$ | |
| 3.173 | Q 37 | O | N | H | $CH_3$ | $OCH_3$ | |
| 3.174 | Q 37 | O | N | H | $OCH_3$ | $N(CH_3)_2$ | |
| 3.175 | Q 37 | O | N | H | $OCH_3$ | $OCH_2CF_3$ | |
| 3.176 | Q 37 | O | N | H | $C_2H_5$ | $OCH_3$ | |
| 3.177 | Q 37 | O | N | H | $OCH_3$ | $OCH_3$ | |
| 3.178 | Q 37 | O | N | H | $CH_3$ | $OC_2H_5$ | |
| 3.179 | Q 37 | O | CH | H | $CH_3$ | $CH_3$ | |
| 3.180 | Q 37 | O | CH | H | $CH_3$ | $OCH_3$ | |
| 3.181 | Q 37 | O | CH | H | $CH_3$ | $OCHF_2$ | |
| 3.182 | Q 37 | O | CH | H | $OCH_3$ | $OCH_3$ | |
| 3.183 | Q 37 | O | CH | H | $OCHF_2$ | $OCHF_2$ | |
| 3.184 | Q 37 | O | CH | H | $OCH_3$ | $OCHF_2$ | |
| 3.185 | Q 38 | O | N | H | $OCH_3$ | $OC_2H_5$ | |
| 3.186 | Q 38 | O | N | H | $OCH_3$ | $OCH(CH_3)_2$ | |
| 3.187 | Q 38 | O | N | H | $CH_3$ | $OCH_3$ | |
| 3.188 | Q 38 | O | N | H | $OCH_3$ | $N(CH_3)_2$ | |
| 3.189 | Q 38 | O | N | H | $OCH_3$ | $OCH_2CF_3$ | |
| 3.190 | Q 38 | O | N | H | $C_2H_5$ | $OCH_3$ | |
| 3.191 | Q 38 | O | N | H | $OCH_3$ | $OCH_3$ | |
| 3.192 | Q 38 | O | N | H | $CH_3$ | $OC_2H_5$ | |
| 3.193 | Q 38 | O | CH | H | $CH_3$ | $CH_3$ | |
| 3.194 | Q 38 | O | CH | H | $CH_3$ | $OCH_3$ | |

Tabelle 3 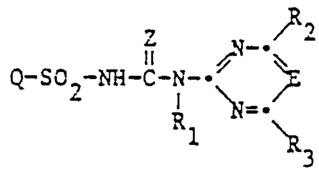 (Fortsetzung)

| Verb. Nr. | Q | Z | E | $R_1$ | $R_2$ | $R_3$ | Smp.[°C] |
|---|---|---|---|---|---|---|---|
| 3.195 | Q 38 | O | CH | H | $CH_3$ | $OCHF_2$ | |
| 3.196 | Q 38 | O | CH | H | $OCH_3$ | $OCH_3$ | |
| 3.197 | Q 38 | O | CH | H | $OCHF_2$ | $OCHF_2$ | |
| 3.198 | Q 38 | O | CH | H | $OCH_3$ | $OCHF_2$ | |
| 3.199 | Q 39 | O | N | H | $OCH_3$ | $OC_2H_5$ | |
| 3.200 | Q 39 | O | N | H | $OCH_3$ | $OCH(CH_3)_2$ | |
| 3.201 | Q 39 | O | N | H | $CH_3$ | $OCH_3$ | |
| 3.202 | Q 39 | O | N | H | $OCH_3$ | $N(CH_3)_2$ | |
| 3.203 | Q 39 | O | N | H | $OCH_3$ | $OCH_2CF_3$ | |
| 3.204 | Q 39 | O | N | H | $C_2H_5$ | $OCH_3$ | |
| 3.205 | Q 39 | O | N | H | $OCH_3$ | $OCH_3$ | |
| 3.206 | Q 39 | O | N | H | $CH_3$ | $OC_2H_5$ | |
| 3.207 | Q 39 | O | CH | H | $CH_3$ | $CH_3$ | |
| 3.208 | Q 39 | O | CH | H | $CH_3$ | $OCH_3$ | |
| 2.209 | Q 39 | O | CH | H | $CH_3$ | $OCHF_2$ | |
| 3.210 | Q 39 | O | CH | H | $OCH_3$ | $OCH_3$ | |
| 3.211 | Q 39 | O | CH | H | $OCHF_2$ | $OCHF_2$ | |
| 3.212 | Q 39 | O | CH | H | $OCH_3$ | $OCHF_2$ | |
| 3.213 | Q 28 | O | N | H | $CH_3$ | $OCH_3$ | |
| 3.214 | Q 28 | O | N | H | $OCH_3$ | $N(CH_3)_2$ | |
| 3.215 | Q 28 | O | N | H | $OCH_3$ | $OC_2H_5$ | |
| 3.216 | Q 28 | O | N | H | $OCH_3$ | $OCH(CH_3)_2$ | |
| 3.217 | Q 28 | O | N | H | $OCH_3$ | $OCH_2CF_3$ | |
| 3.218 | Q 28 | O | N | H | $C_2H_5$ | $OCH_3$ | |
| 3.219 | Q 28 | O | N | H | $OC_2H_5$ | $OC_2H_5$ | |
| 3.220 | Q 28 | O | N | H | $SCH_3$ | $OCH_3$ | |
| 3.221 | Q 28 | O | N | H | $CH_3$ | $OC_2H_5$ | |

Tabelle 3 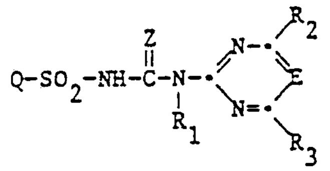 (Fortsetzung)

| Verb. Nr. | Q | Z | E | $R_1$ | $R_2$ | $R_3$ | Smp. [°C] |
|---|---|---|---|---|---|---|---|
| 3.222 | Q 28 | O | CH | H | $CH_3$ | $CH_3$ | |
| 3.223 | Q 28 | O | CH | H | $CH_3$ | $OCH_3$ | |
| 3.224 | Q 28 | O | CH | H | $CH_3$ | $OCHF_2$ | |
| 3.225 | Q 28 | O | CH | H | $CH_3$ | $N(CH_3)_2$ | |
| 3.226 | Q 28 | O | CH | H | $OCH_3$ | $OCH_3$ | |
| 2.227 | Q 28 | O | CH | H | $OCHF_2$ | $OCHF_2$ | |
| 3.228 | Q 28 | O | CH | H | $OCH_3$ | $OCHF_2$ | |
| 3.229 | Q 28 | O | CH | H | $OCH_3$ | Cl | |
| 3.230 | Q 28 | O | CH | H | $OCH_3$ | F | |
| 3.231 | Q 28 | O | CH | H | $OCH_3$ | $N(CH_3)_2$ | |
| 3.232 | Q 28 | O | CH | H | $CH_2F$ | $OCH_3$ | |
| 3.233 | Q 28 | O | CH | H | $OCHF_2$ | $N(CH_3)_2$ | |
| 3.234 | Q 28 | O | CH | H | $OCH_3$ | $OCF_2-CHF_2$ | |
| 3.235 | Q 28 | O | CH | H | $CH_3$ | $OCF_2-CHF_2$ | |
| 3.236 | Q 29 | O | N | H | $CH_3$ | $OCH_3$ | |
| 3.237 | Q 29 | O | N | H | $OCH_3$ | $N(CH_3)_2$ | |
| 3.238 | Q 29 | O | N | H | $OCH_3$ | $OC_2H_5$ | |
| 3.239 | Q 29 | O | N | H | $OCH_3$ | $OCH(CH_3)_2$ | |
| 3.240 | Q 29 | O | N | H | $OCH_3$ | $OCH_2CF_3$ | |
| 3.241 | Q 29 | O | N | H | $C_2H_5$ | $OCH_3$ | |
| 3.242 | Q 29 | O | N | H | $OC_2H_5$ | $OC_2H_5$ | |
| 3.243 | Q 29 | O | N | H | $SCH_3$ | $OCH_3$ | |
| 3.244 | Q 29 | O | N | H | $CH_3$ | $OC_2H_5$ | |
| 3.245 | Q 29 | O | CH | H | $CH_3$ | $CH_3$ | |
| 3.246 | Q 29 | O | CH | H | $CH_3$ | $OCH_3$ | |
| 3.247 | Q 29 | O | CH | H | $CH_3$ | $OCHF_2$ | |
| 3.248 | Q 29 | O | CH | H | $CH_3$ | $N(CH_3)_2$ | |
| 3.249 | Q 29 | O | CH | H | $OCH_3$ | $OCH_3$ | |

Tabelle 3 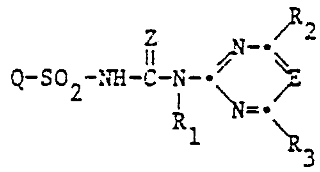 (Fortsetzung)

| Verb. Nr. | Q | Z | E | $R_1$ | $R_2$ | $R_3$ | Smp. [°C] |
|---|---|---|---|---|---|---|---|
| 3.250 | Q 29 | O | CH | H | $OCHF_2$ | $OCHF_2$ | |
| 3.251 | Q 29 | O | CH | H | $OCH_3$ | $OCHF_2$ | |
| 3.252 | Q 29 | O | CH | H | $OCH_3$ | Cl | |
| 3.253 | Q 29 | O | CH | H | $OCH_3$ | F | |
| 3.254 | Q 29 | O | CH | H | $OCH_3$ | $N(CH_3)_2$ | |
| 3.255 | Q 29 | O | CH | H | $CH_2F$ | $OCH_3$ | |
| 3.256 | Q 29 | O | CH | H | $OCHF_2$ | $N(CH_3)_2$ | |
| 3.257 | Q 29 | O | CH | H | $OCH_3$ | $OCF_2-CHF_2$ | |
| 3.258 | Q 29 | O | CH | H | $CH_3$ | $OCF_2-CHF_2$ | |
| 3.259 | Q 31 | O | N | H | $CH_3$ | $OCH_3$ | |
| 3.260 | Q 31 | O | N | H | $OCH_3$ | $N(CH_3)_2$ | |
| 3.261 | Q 31 | O | N | H | $OCH_3$ | $OC_2H_5$ | |
| 3.262 | Q 31 | O | N | H | $OCH_3$ | $OCH(CH_3)_2$ | |
| 3.263 | Q 31 | O | N | H | $OCH_3$ | $OCH_2CF_3$ | |
| 3.264 | Q 31 | O | N | H | $C_2H_5$ | $OCH_3$ | |
| 3.265 | Q 31 | O | N | H | $OC_2H_5$ | $OC_2H_5$ | |
| 3.266 | Q 31 | O | N | H | $SCH_3$ | $OCH_3$ | |
| 3.267 | Q 31 | O | N | H | $CH_3$ | $OC_2H_5$ | |
| 3.268 | Q 31 | O | CH | H | $CH_3$ | $CH_3$ | |
| 3.269 | Q 31 | O | CH | H | $CH_3$ | $OCH_3$ | |
| 3.270 | Q 31 | O | CH | H | $CH_3$ | $OCHF_2$ | |
| 3.271 | Q 31 | O | CH | H | $CH_3$ | $N(CH_3)_2$ | |
| 3.272 | Q 31 | O | CH | H | $OCH_3$ | $OCH_3$ | |
| 3.273 | Q 31 | O | CH | H | $OCHF_2$ | $OCHF_2$ | |
| 3.274 | Q 31 | O | CH | H | $OCH_3$ | $OCHF_2$ | |
| 3.275 | Q 31 | O | CH | H | $OCH_3$ | Cl | |
| 3.276 | Q 31 | O | CH | H | $OCH_3$ | F | |
| 3.277 | Q 31 | O | CH | H | $OCH_3$ | $N(CH_3)_2$ | |

Tabelle 3 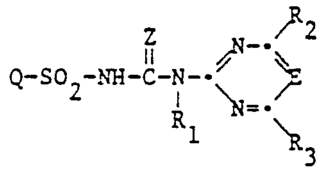 (Fortsetzung)

| Verb. Nr. | Q | Z | E | $R_1$ | $R_2$ | $R_3$ | Smp. [°C] |
|---|---|---|---|---|---|---|---|
| 3.278 | Q 31 | O | CH | H | $CH_2F$ | $OCH_3$ | |
| 3.279 | Q 31 | O | CH | H | $OCHF_2$ | $N(CH_3)_2$ | |
| 3.280 | Q 31 | O | CH | H | $OCH_3$ | $OCF_2-CHF_2$ | |
| 3.281 | Q 31 | O | CH | H | $CH_3$ | $OCF_2-CHF_2$ | |
| 3.282 | Q 35 | O | N | H | $CH_3$ | $OCH_3$ | |
| 3.283 | Q 35 | O | N | H | $OCH_3$ | $N(CH_3)_2$ | |
| 3.284 | Q 35 | O | N | H | $OCH_3$ | $OC_2H_5$ | |
| 3.285 | Q 35 | O | N | H | $OCH_3$ | $OCH(CH_3)_2$ | |
| 3.286 | Q 35 | O | N | H | $OCH_3$ | $OCH_2CF_3$ | |
| 3.287 | Q 35 | O | N | H | $C_2H_5$ | $OCH_3$ | |
| 3.288 | Q 35 | O | N | H | $OC_2H_5$ | $OC_2H_5$ | |
| 3.289 | Q 35 | O | N | H | $SCH_3$ | $OCH_3$ | |
| 3.290 | Q 35 | O | N | H | $CH_3$ | $OC_2H_5$ | |
| 3.291 | Q 35 | O | CH | H | $CH_3$ | $CH_3$ | |
| 3.292 | Q 35 | O | CH | H | $CH_3$ | $OCH_3$ | |
| 3.293 | Q 35 | O | CH | H | $CH_3$ | $OCHF_2$ | |
| 3.294 | Q 35 | O | CH | H | $CH_3$ | $N(CH_3)_2$ | |
| 3.295 | Q 35 | O | CH | H | $OCH_3$ | $OCH_3$ | |
| 3.296 | Q 35 | O | CH | H | $OCHF_2$ | $OCHF_2$ | |
| 3.297 | Q 35 | O | CH | H | $OCH_3$ | $OCHF_2$ | |
| 3.298 | Q 35 | O | CH | H | $OCH_3$ | Cl | |
| 3.299 | Q 35 | O | CH | H | $OCH_3$ | F | |
| 3.300 | Q 35 | O | CH | H | $OCH_3$ | $N(CH_3)_2$ | |
| 3.301 | Q 35 | O | CH | H | $CH_2F$ | $OCH_3$ | |
| 3.303 | Q 35 | O | CH | H | $OCHF_2$ | $N(CH_3)_2$ | |
| 3.304 | Q 35 | O | CH | H | $OCH_3$ | $OCF_2-CHF_2$ | |
| 3.305 | Q 35 | O | CH | H | $CH_3$ | $OCF_2-CHF_2$ | |
| 3.306 | Q 1 | O | N | H | $OCH_3$ | $OCH_3$ | 142-143 |

Tabelle 3 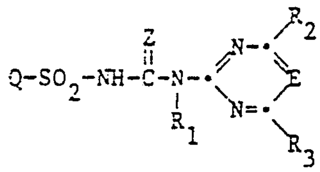 (Fortsetzung)

| Verb. Nr. | Q | Z | E | $R_1$ | $R_2$ | $R_3$ | Smp. [°C] |
|---|---|---|---|---|---|---|---|
| 3.307 | Q 2 | O | N | H | $OCH_3$ | $OCH_3$ | 124-126 |
| 3.308 | Q 36 | O | N | H | $CH_3$ | $OCH_3$ | |
| 3.309 | Q 36 | O | N | H | $OCH_3$ | $N(CH_3)_2$ | |
| 3.310 | Q 36 | O | N | H | $OCH_3$ | $OC_2H_5$ | |
| 3.311 | Q 36 | O | N | H | $OCH_3$ | $OCH(CH_3)_2$ | |
| 3.312 | Q 36 | O | N | H | $OCH_3$ | $OCH_2CF_3$ | |
| 3.313 | Q 36 | O | N | H | $C_2H_5$ | $OCH_3$ | |
| 3.314 | Q 36 | O | N | H | $OC_2H_5$ | $OC_2H_5$ | |
| 3.315 | Q 36 | O | N | H | $SCH_3$ | $OCH_3$ | |
| 3.316 | Q 36 | O | N | H | $CH_3$ | $OC_2H_5$ | |
| 3.317 | Q 36 | O | CH | H | $CH_3$ | $CH_3$ | |
| 3.318 | Q 36 | O | CH | H | $CH_3$ | $OCH_3$ | |
| 3.319 | Q 36 | O | CH | H | $CH_3$ | $OCHF_2$ | |
| 3.320 | Q 36 | O | CH | H | $CH_3$ | $N(CH_3)_2$ | |
| 3.321 | Q 36 | O | CH | H | $OCH_3$ | $OCH_3$ | |
| 3.322 | Q 36 | O | CH | H | $OCHF_2$ | $OCHF_2$ | |
| 3.323 | Q 36 | O | CH | H | $OCH_3$ | $OCHF_2$ | |
| 3.324 | Q 36 | O | CH | H | $OCH_3$ | Cl | |
| 3.325 | Q 36 | O | CH | H | $OCH_3$ | F | |
| 3.326 | Q 36 | O | CH | H | $OCH_3$ | $N(CH_3)_2$ | |
| 3.327 | Q 36 | O | CH | H | $CH_2F$ | $OCH_3$ | |
| 3.328 | Q 36 | O | CH | H | $OCHF_2$ | $N(CH_3)_2$ | |
| 3.329 | Q 36 | O | CH | H | $OCH_3$ | $OCF_2-CHF_2$ | |
| 3.330 | Q 36 | O | CH | H | $CH_3$ | $OCF_2-CHF_2$ | |

Zur Applikation können die Verbindungen der Formel I in den folgenden Aufarbeitungsformen vorliegen.

Formulierungsbeispiele

Beispiel 2: Formulierungsbeispiele für Wirkstoffe der Formel I
( % = Gewichtsprozent)

| a) Spritzpulver | a) | b) | c) |
|---|---|---|---|
| Wirkstoff | 20% | 60% | 0,5% |
| Na-Ligninsulfonat | 5% | 5% | 5% |
| Na-Laurylsulfat | 3% | - | - |
| Na-Diisobutylnaphthalinsulfonat | - | 6% | 6% |
| Octylphenolpolyäthylenglykoläther (7-8 Mol AeO) | - | 2% | 2% |
| Hochdisperse Kieselsäure | 5% | 27% | 27% |
| Kaolin | 67% | - | - |
| Natriumchlorid | - | - | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsion-Konzentrat | a) | b) |
|---|---|---|
| Wirkstoff | 10% | 1% |
| Octylphenolpolyäthylenglykoläther (4-5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 0,1% | 1% |
| Talkum | 99,9% | - |
| Kaolin | - | 99% |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff | 40% | 5% |
| Aethylenglykol | 10% | 10% |
| Nonylphenolpolyäthylenglykoläther (15 Mol) AeO) | 6% | 1% |
| Na-Ligninsulfonat | 10% | 5% |
| Carboxymethylcellulose | 1% | 1% |
| 37%-ige wässrige Formaldehyd-Lösung | 0,2% | 0,2% |
| Silikonöl in Form einer 75%-igen wässrigen Emulsion | 0,8% | 0,8% |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff | 5% |
| Isopropylamin | 1% |
| Octylphenolpolyäthylenglykoläther (78 Mol AeO) | 3% |
| Wasser | 91%. |

Aus solchen Konzentraten können durch Verdünnen mit Wasser Emulsionen der gewünschten Anwendungskonzentration hergestellt werden, die besonders zur Blattapplikation geeignet sind. Darüberhinaus können weitere Spritzpulver mit anderen Mischungsverhältnissen oder anderen in der Formulierungstechnik gebräuchlichen Trägermaterialien und Zuschlagstoffen hergestellt werden. Die Wirkstoffe werden in geeigneten Mischern mit den genannten Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnt und insbesondere zur Blattapplikation verwenden lassen. Solche Mittel gehören ebenfalls zur Erfindung.

Mittel, die nach der oben beschriebenen Art formuliert wurden und als Wirkungskomponente eine der Verbindungen aus der Tabelle 1 enthielten, liessen sich mit sehr gutem Erfolg pflanzenwuchsregulatorisch und/oder herbizid einsetzen.

## Biologische Beispiele

### Beispiel 3 : Nachweis der Herbizidwirkung vor dem Auflaufen der Pflanzen

Im Gewächshaus werden Pflanzensamen in Blumentöpfe von 12-15 cm Durchmesser gesät. Unittelbar danach wird die Erdoberfläche mit einer wässrigen Dispersion oder Lösung der Wirkstoffe behandelt. Es werden Konzentrationen von 4 kg Wirkstoffmengen pro Hektar angewendet. Die Töpfe

werden dann im Gewächshaus bei einer Temperatur von 22-25°C und 50-70 % relativer Luftfeuchtigkeit gehalten. Nach 3 Wochen wird der Versuch ausgewertet. Die Verbindungen der Formel I zeigen in diesem Versuch gute Wirkung, d. h. die Versuchspflanzen werden irreversibel geschädigt, so dass sie absterben oder verkümmern.

Beispiel 4 : Nachweis der Herbizidwirkung nach dem Auflaufen der Pflanzen (Kontaktwirkung)

Eine grössere Anzahl Unkräuter und Kulturpflanzen, sowohl monokotyle wie dikotyle, werden nach dem Auflaufen, im 4- bis 6-Blattstadium mit einer wässrigen Wirkstoffdispersion in einer Dosierung von 4 kg AS/ha auf die Pflanzen gespritzt und diese bei 24° bis 26°C und 45-60 % relativer Luftfeuchtigkeit gehalten. 15 Tage nach der Behandlung zeigen die Verbindungen der Formel I gute Herbizidwirkung.

Beispiel 5 : Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21 Tage nach der Aussat mit der wässrigen Spritzbrühe eines Wirkstoffes der Formel I besprüht. Die Wirkstoffmenge beträgt umgerechnet 0,5 bzw. 2,5 kg Aktivsubstanz pro Hektar. 10 und 21 Tage nach Applikation wird das Wachstum des Getreides beurteilt. Hierbei kann festgestellt werden, dass Getreidepflanzen die mit Wirkstoffen der Formel I behandelt worden sind, im Vergleich zu unbehandelten Kontrollpflanzen eine starke Wuchsreduktion aufweisen.

Beispiel 6 : Wuchshemmung bei Gräsern

In Kunststoffschalen mit Erde-Topf-Sand-Gemisch (6 :3 :1) werden die Gräser Lolium perenne, Poa pratensis, Festuca ovina und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet 2,5 kg Aktivsubstanz pro Hektar. 10 und 21 Tage nach Applikation wird das Wachstum der Gräser beurteilt, dabei zeigt es sich, dass die erfindungsgemässen Wirkstoffe eine merkliche Wuchshemmung bewirken.

**Patentansprüche** (für die Vertragsstaaten : BE, CH, DE, FR, GB, IT, LI, NL)

1. Verbindungen der Formel I

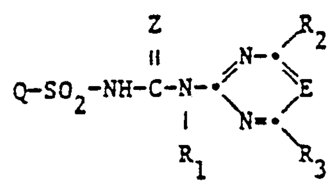

(I)

worin

$R_1$ für Wasserstoff oder $C_1$-$C_5$-Alkyl bedeutet ;

$R_2$ und $R_3$ unabhängig voneinander Wasserstoff, $C_1$-$C_5$-Alkyl, $C_1$-$C_5$-Alkoxy, $C_1$-$C_5$-Alkylthio, $C_1$-$C_5$-Haloalkyl, Halogen, $C_1$-$C_5$-Halogenalkoxy, $C_1$-$C_5$-Halogenalkylthio, $C_1$-$C_4$-Alkylamino, Di-$C_1$-$C_4$-alkylamino, eine Alkoxyalkylgruppe oder Alkoxyalkoxygruppe mit maximal 6 Kohlenstoffatomen bedeuten ;

Z für Sauerstoff oder Schwefel steht ;

E für —CH= oder —N= steht und

Q einen über ein Kohlenstoffatom gebundenen, gegebenenfalls durch Halogen, Pseudohalogen, Nitro, $C_1$-$C_6$-Alkyl, $C_1$-$C_6$-Halogenalkyl, $C_1$-$C_6$-Alkoxy, $C_1$-$C_6$-Alkylthio, $C_1$-$C_6$-Halogenalkoxy, $C_1$-$C_6$-Halogenalkylthio, Amino, $C_1$-$C_6$-Alkylamino, Di-$C_1$-$C_6$-alkylamino, $C_1$-$C_6$-Alkylcarbonylamino, $C_1$-$C_6$-Alkylcarbonyl, $C_1$-$C_6$-Alkoxycarbonyl, $C_1$-$C_6$-Alkylthiocarbonyl, Carbamoyl, $C_1$-$C_6$-Alkylaminocarbonyl, Di-$C_1$-$C_6$-alkylaminocarbonyl, $C_1$-$C_6$-Alkylsulfinyl, $C_1$-$C_6$-Alkylsulfonyl, $C_2$-$C_6$-Alkenyloxy, $C_3$-$C_6$-Alkinyloxy oder ein gegebenenfalls durch Halogen, Nitro, Cyano, $C_1$-$C_6$-Alkyl, $C_1$-$C_6$-Alkoxy, $C_1$-$C_6$-Halogenalkyl, $C_1$-$C_6$-Halogenalkoxy substituiertes Phenyl, Phenoxy, Phenylthio oder eine gegebenenfalls durch Halogen und/oder $C_1$-$C_6$-Alkylsubstituierte Benzylgruppe substituierten Pyrimidin-, Pyridazin-, Pyrazin- oder Triazin-Rest bedeutet, sowie die Salze dieser Verbindungen.

2. Verbindungen der Formel I, nach Anspruch 1, dadurch gekennzeichnet, dass Z für Sauerstoff und $R_1$ für Wasserstoff steht.

3. Verbindungen der Formel I nach Anspruch 1, dadurch gekennzeichnet, dass $R_1$ für Wasserstoff steht, $R_2$ und $R_3$ unabhängig voneinander $C_1$-$C_3$-Alkyl, $C_1$-$C_3$-Alkoxy, $C_1$-$C_3$-Halogenalkyl, $C_1$-$C_3$-Halogenalkoxy, Halogen oder $C_2$-$C_4$-Alkoxyalkyl, Z Sauerstoff, E Stickstoff oder CH bedeuten, Q für einen

unter Formel I definierten heterocyclischen Rest steht, der gegebenenfalls durch Chlor, Brom, Fluor, Nitro, Cyano, $C_1$-$C_4$-Alkylcarbonyl, $C_3$-$C_5$-Alkoxyalkoxy, $C_1$-$C_4$-Alkyloxycarbonyl, $C_1$-$C_3$-Alkoxy, $C_1$-$C_3$-Alkylthio, $C_1$-$C_3$-Alkylsulfinyl, $C_1$-$C_3$-Alkylsulfonyl, $C_1$-$C_4$-Alkyl, Trifluormethyl, Trichlormethyl, Cyano-$C_1$-$C_4$-Alkyl, $C_2$-$C_4$-Alkenyloxy, $C_3$-$C_4$-Alkinyloxy oder N-$C_1$-$C_4$-Alkylcarbonylamino substituiert ist.

4. Verbindungen gemäss Anspruch 3, dadurch gekennzeichnet, dass Q für einen Pyrazinylrest steht.

5. N-(2-Methyl-pyrazin-3-ylsulfonyl)-N'-(4-methoxy-6-methyl-1,3,5-triazin-2-yl)-harnstoff gemäss Anspruch 1.

6. N-(2-Methoxy-pyrazin-3-ylsulfonyl)-N'-(4-methoxy-6-methyl-1,3,5-triazin-2-yl)-harnstoff gemäss Anspruch 1.

7. Verfahren zur Herstellung von Verbindungen der Formel I, dadurch gekennzeichnet, dass man ein Phenylsulfonylderivat der Formel II

$$Q—SO_2W \qquad (II)$$

mit einer Verbindung der Formel III

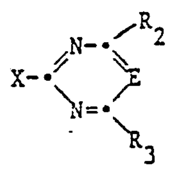

(III)

umsetzt, wobei X und W für eine der Gruppen $—NH_2$, $—N=C=Z$ oder $—NR_1—CZ—OR$ stehen ; die Substituenten $R_1$, $R_2$, $R_3$, Q, Z und E die unter Formel I angegebenen Bedeutungen haben, R für einen aliphatischen oder aromatischen Rest steht ; mit der Massgabe, dass die Reaktionspartner der Formeln II und III stets so gewählt werden, dass eine Aminofunktion entweder mit einer Isocyanato-, Isothiocyanato- oder mit einer [$—NR_1—CZ—OR$]-Gruppe zur Reaktion gelangt.

8. Verfahren nach Anspruch 7, dadurch gekennzeichnet, dass R für $C_1$-$C_4$-Alkyl, Phenyl oder Benzyl steht.

9. Verfahren nach Anspruch 7, dadurch gekennzeichnet, dass man in Gegenwart eines reaktionsinerten Lösungsmittels oder eines Lösungsmittelgemisches arbeitet.

10. Verfahren nach Anspruch 9, dadurch gekennzeichnet, dass die Reaktion in Gegenwart einer Base durchgeführt wird.

11. Verbindungen der Formel

$$Q—SO_2—NH—CZ—OR$$

worin Q und Z die unter Formel I in Anspruch 1 und R die unter Formel II in Anspruch 7 gegebenen Bedeutungen haben.

12. Verbindungen der Formel

$$Q—SO_2—NH_2$$

worin Q die unter Formel I in Anspruch 1 gegebene Bedeutung hat, mit der Massgabe, dass Q nicht für 4-Methyl-2-pyrimidinyl, 5-Methyl-2-pyrimidinyl, 2,4-Dimethoxy-6-pyrimidinyl, 4-Methoxy-6-pyrimidinyl, 3-Amino-2-pyrazinyl, 2-Amino-5-methyl-3-pyrazinyl, 3-Amino-5,6-dimethyl-2-pyrazinyl oder 4,6-Dimethyl-2-pyrimidinyl steht.

13. Herbizides und/oder pflanzenwuchsregulierendes Mittel, dadurch gekennzeichnet, dass es als mindestens eine aktive Komponente eine Verbindung der Formel I nach Anspruch 1 enthält.

14. Mittel nach Anspruch 13, dadurch gekennzeichnet, dass es 0,01 bis 95 Gewichtsprozent einer Verbindung der Formel I nach Anspruch 1 und 1 bis 99,99 Gewichtsprozent an Zuschlagstoffen, darunter 0 bis 25 Gewichtsprozent eines Tensides enthält.

15. Verwendung einer Verbindung der Formel I nach Anspruch 1 zur Bekämpfung von unerwünschten Pflanzensorten in Kulturen von Nutzpflanzen und/oder zur Regulierung des Pflanzenwachstums von Nutzpflanzen.

16. Verwendung nach Anspruch 15 zur selektiven Unkrautbekämpfung.

17. Verwendung nach Anspruch 15 zur Hemmung des Pflanzenwuchses.

18. Verfahren zur pre- oder post-emergenten Bekämpfung von Unkräutern und/oder zur Beeinflussung des Wachstums von Nutzpflanzen, dadurch gekennzeichnet, dass man eine wirksame Menge eines Wirkstoffes der Formel I gemäss Anspruch 1 auf die Schad- oder Nutzpflanze, deren Pflanzenteile oder deren Standort appliziert.

**Patentansprüche** (für den Vertraagsstaat AT)

1. Herbizides und/oder pflanzenwuchsregulierendes Mittel, dadurch gekennzeichnet, dass es als mindestens eine aktive Komponente eine Verbindung der Formel I

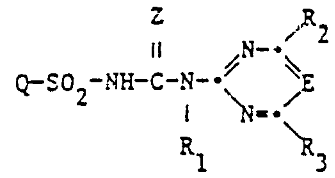

(I)

oder ein Salz davon enthält, worin

$R_1$ für Wasserstoff oder $C_1$-$C_5$-Alkyl bedeutet ;

$R_2$ und $R_3$ unabhängig voneinander Wasserstoff, $C_1$-$C_5$-Alkyl, $C_1$-$C_5$-Alkoxy, $C_1$-$C_5$-Alkylthio, $C_1$-$C_5$-Haloalkyl, Halogen, $C_1$-$C_5$-Halogenalkoxy, $C_1$-$C_5$-Halogenalkylthio, $C_1$-$C_4$-Alkylamino, Di-$C_1$-$C_4$-alkylamino, eine Alkoxyalkylgruppe oder Alkoxyalkoxygruppe mit maximal 6 Kohlenstoffatomen bedeuten ;

Z für Sauerstoff oder Schwefel steht ;

E für —CH= oder —N= steht und

Q einen über ein Kohlenstoffatom gebundenen, gegebenenfalls durch Halogen, Pseudohalogen, Nitro, $C_1$-$C_6$-Alkyl, $C_1$-$C_6$-Halogenalkyl, $C_1$-$C_6$-Alkoxy, $C_1$-$C_6$-Alkylthio, $C_1$-$C_6$-Halogenalkoxy, $C_1$-$C_6$-Halogenalkylthio, Amino, $C_1$-$C_6$-Alkylamino, Di-$C_1$-$C_6$-alkylamino, $C_1$-$C_6$-Alkylcarbonylamino, $C_1$-$C_6$-Alkylcarbonyl, $C_1$-$C_6$-Alkoxycarbonyl, $C_1$-$C_6$-Alkylthiocarbonyl, Carbamoyl, $C_1$-$C_6$-Alkylaminocarbonyl, Di-$C_1$-$C_6$-alkylaminocarbonyl, $C_1$-$C_6$-Alkylsulfinyl, $C_1$-$C_6$-Alkylsulfonyl, $C_2$-$C_6$-Alkenyloxy, $C_3$-$C_6$-Alkinyloxy oder ein gegebenenfalls durch Halogen, Nitro, Cyano, $C_1$-$C_6$-Alkyl, $C_1$-$C_6$-Alkoxy, $C_1$-$C_6$-Halogenalkyl, $C_1$-$C_6$-Halogenalkoxy substituiertes Phenyl, Phenoxy, Phenylthio oder eine gegebenenfalls durch Halogen und/oder $C_1$-$C_6$-Alkyl substituierte Benzylgruppe substituierten Pyrimidin-, Pyridazin-, Pyrazin- oder Triazin-Rest bedeutet.

2. Mittel gemäss Anspruch 1, dadurch gekennzeichnet, dass Z für Sauerstoff und $R_1$ für Wasserstoff steht.

3. Mittel gemäss Anspruch 1, dadurch gekennzeichnet, dass $R_1$ für Wasserstoff steht, $R_2$ und $R_3$ unabhängig voneinander $C_1$-$C_3$-Alkyl, $C_1$-$C_3$-Alkoxy, $C_1$-$C_3$-Halogenalkyl, $C_1$-$C_3$-Halogenalkoxy, Halogen oder $C_2$-$C_4$-Alkoxyalkyl, Z Sauerstoff, E Stickstoff oder CH bedeuten, Q für einen unter Formel I definierten heterocyclischen Rest steht, der gegebenenfalls durch Chlor, Brom, Fluor, Nitro, Cyano, $C_1$-$C_4$-Alkylcarbonyl, $C_3$-$C_5$-Alkoxyalkoxy, $C_1$-$C_4$-Alkyloxycarbonyl, $C_1$-$C_3$-Alkoxy, $C_1$-$C_3$-Alkylthio, $C_1$-$C_3$-Alkylsulfinyl, $C_1$-$C_3$-Alkylsulfonyl, $C_1$-$C_4$-Alkyl, Trifluormethyl, Trichlormethyl, Cyano-$C_1$-$C_4$-Alkyl, $C_2$-$C_4$-Alkenyloxy, $C_3$-$C_4$-Alkinyloxy oder N-$C_1$-$C_4$-Alkylcarbonylamino substituiert ist.

4. Mittel gemäss Anspruch 3, dadurch gekennzeichnet, dass Q für einen Pyrazinrest steht.

5. Mittel gemäss Anspruch 1, dadurch gekennzeichnet, dass es als Wirkstoff N-(2-Methyl-pyrazin-3-ylsulfonyl)-N'-(4-methoxy-6-methyl-1,3,5-triazin-2-yl)-harnstoff enthält.

6. Mittel gemäss Anspruch 1, dadurch gekennzeichnet, dass es als Wirkstoff N-(2-Methoxy-pyrazin-3-ylsulfonyl)-N'-(4-methoxy-6-methyl-1,3,5-triazin-2-yl)-harnstoff enthält.

7. Verfahren zur Herstellung von Verbindungen der Formel I, dadurch gekennzeichnet, dass man ein Phenylsulfonylderivat der Formel II

$$Q—SO_2W \qquad (II)$$

mit einer Verbindung der Formel III

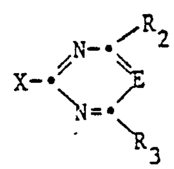

(III)

umsetzt, wobei X und W für eine der Gruppen $—NH_2$, —N=C=Z oder $—NR_1—CZ—OR$ stehen ; die Substituenten $R_1$, $R_2$, $R_3$, Q, Z und E die unter Formel I angegebenen Bedeutungen haben, R für einen aliphatischen oder aromatischen Rest steht ; mit der Massgabe, dass die Reaktionspartner der Formeln II und III stets so gewählt werden, dass eine Aminofunktion entweder mit einer Isocyanato-, Isothiocyanato- oder mit einer [$—NR_1$ —CZ—OR]-Gruppe zur Reaktion gelangt.

8. Verfahren nach Anspruch 7, dadurch gekennzeichnet, dass R für $C_1$-$C_4$-Alkyl, Phenyl oder Benzyl steht.

9. Verfahren nach Anspruch 7, dadurch gekennzeichnet, dass man in Gegenwart eines reaktionsinerten Lösungsmittels oder eines Lösungsmittelgemisches arbeitet.

10. Verfahren nach Anspruch 9, dadurch gekennzeichnet, dass die Reaktion in Gegenwart einer Base durchgeführt wird.

11. Mittel nach Anspruch 1, dadurch gekennzeichnet, dass es 0,01 bis 95 Gewichtsprozent einer Verbindung der Formel I nach Anspruch 1 und 1 bis 99,99 Gewichtsprozent an Zuschlagstoffen, darunter 0 bis 25 Gewichtsprozent eines Tensides enthält.

12. Verwendung einer Verbindung der Formel I nach Anspruch 1 zur Bekämpfung von unerwünschten Pflanzensorten in Kulturen von Nutzpflanzen und/oder zur Regulierung des Pflanzenwachstums von Nutzpflanzen.

13. Verwendung nach Anspruch 12 zur selektiven Unkrautbekämpfung.

14. Verwendung nach Anspruch 12 zur Hemmung des Pflanzenwuchses.

15. Verfahren zur pre- oder post-emergenten Bekämpfung von Unkräutern und/oder zur Beeinflussung des Wachstums von Nutzpflanzen, dadurch gekennzeichnet, dass man eine wirksame Menge eines Wirkstoffes der Formel I gemäss Anspruch 1 auf die Schad- oder Nutzpflanze, deren Pflanzenteile oder deren Standort appliziert.

**Claims** (for the Contracting States : BE, CH, DE, FR, GB, IT, LI, NL)

1. Compounds of the formula

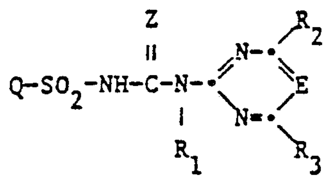

(I)

wherein

$R_1$ is hydrogen or $C_1$-$C_5$alkyl, each of

$R_2$ and $R_3$, independently of the other, is hydrogen, $C_1$-$C_5$alkyl, $C_1$-$C_5$alkoxy, $C_1$-$C_5$alylthio, $C_1$-$C_5$haloalkyl, halogen, $C_1$-$C_5$haloaloxy, $C_1$-$C_5$haloalkylthio, $C_1$-$C_4$alkylamino, di-$C_1$-$C_4$alkylamino, or an alkoxyalkyl group or alkoxyalkoxy group containing not more than 6 carbon atoms,

Z is oxygen or sulfur,

E is —CH= or —N=, and

Q is a pyrimidine, pyridazine, pyrazine or triazine radical that is bonded by way of a carbon atom and is unsubstituted or substituted by halogen, pseudohalogen, nitro, $C_1$-$C_6$alkyl, $C_1$-$C_6$haloalkyl, $C_1$-$C_6$alkoxy, $C_1$-$C_6$alkylthio, $C_1$-$C_6$haloalkoxy, $C_1$-$C_6$haloalkylthio, amino, $C_1$-$C_6$alkylamino, di-$C_1$-$C_6$alkylamino, $C_1$-$C_6$alkylcarbonylamino, $C_1$-$C_6$alkylcarbonyl, $C_1$-$C_6$alkoxycarbonyl, $C_1$-$C_6$alkylthiocarbonyl, carbamoyl, $C_1$-$C_6$alkylaminocarbonyl, di-$C_1$-$C_6$alkylaminocarbonyl, $C_1$-$C_6$alkylsulfinyl, $C_1$-$C_6$alkylsulfonyl, $C_2$-$C_6$alkenyloxy, $C_3$-$C_6$-alkynyloxy, or by phenyl, phenoxy or phenylthio each of which is unsubstituted or substituted by halogen, nitro, cyano, $C_1$-$C_6$alkyl, $C_1$-$C_6$alkoxy, $C_1$-$C_6$haloalkyl or by $C_1$-$C_6$haloalkoxy, or by a benzyl group that is unsubstituted or substituted by halogen and/or $C_1$-$C_6$alkyl, and salts of these compounds.

2. Compounds of the formula I according to claim 1 wherein Z is oxygen and $R_1$ is hydrogen.

3. Compounds of the formula I according to claim 1 wherein $R_1$ is hydrogen, each of $R_2$ and $R_3$, independently of the other, is $C_1$-$C_3$alkyl, $C_1$-$C_3$alkoxy, $C_1$-$C_3$haloalkyl, $C_1$-$C_3$haloalkoxy, halogen or $C_2$-$C_4$alkoxyalkyl, Z is oxygen, E is nitrogen or CH, and Q is a heterocyclic radical as defined for formula I that is unsubstituted or substituted by chlorine, bromine, fluorine, nitro, cyano, $C_1$-$C_4$alkylcarbonyl, $C_3$-$C_5$alkoxyalkoxy, $C_1$-$C_4$alkoxycarbonyl, $C_1$-$C_3$-alkoxy, $C_1$-$C_3$alkylthio, $C_1$-$C_3$alkylsulfinyl, $C_1$-$C_3$alkylsulfonyl, $C_1$-$C_4$alkyl, trifluoromethyl, trichloromethyl, cyano-$C_1$-$C_4$alkyl, $C_2$-$C_4$alkenyloxy, $C_3$-$C_4$alkynyloxy or by N-$C_1$-$C_4$alkylcarbonylamino.

4. Compounds according to claim 3 wherein Q is a pyrazinyl radical.

5. N-(2-methylpyrazin-3-ylsulfonyl)-N'-(4-methoxy-6-methyl-1,3,5-triazin-2-yl)-urea according to claim 1.

6. N-(2-methoxypyrazin-3-ylsulfonyl)-N'-(4-methoxy-6-methyl-1,3,5-triazin-2-yl)-urea according to claim 1.

7. A process for the preparation of compounds of the formula I, which comprises reacting a phenylsulfonyl derivative of the formula II

$$Q—SO_2W \qquad (II)$$

with a compound of the formula III

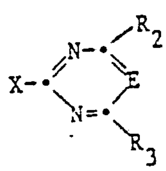

(III)

wherein X and W are one of the groups $—NH_2$, $—N=C=Z$ or $—NR_1—CZ—OR$, the substituents $R_1$, $R_2$, $R_3$, Q, Z and E are as defined for formula I, and R is an aliphatic or aromatic radical, with the proviso that the

reactants of the formulae II and III are always so selected that an amino function reacts either with an isocyanato or isothiocyanato group or with an [—$NR_1$—CZ—OR] group.

8. A process according to claim 7 wherein R is $C_1$-$C_4$alkyl, phenyl or benzyl.

9. A process according to claim 7 wherein the reaction is carried out in the presence of a solvent that is inert towards the reaction or a solvent mixture.

10. A process according to claim 9 wherein the reaction is carried out in the presence of a base.

11. Compounds of the formula

$$Q—SO_2—NH—CZ—OR$$

wherein Q and Z are as defined for formula I in claim 1 and R is as defined for formula II in claim 7.

12. Compounds of the formula

$$Q—SO_2—NH_2$$

wherein Q is as defined for formula I in claim 1, with the proviso that Q is not 4-methyl-2-pyrimidinyl, 5-methyl-2-pyrimidinyl, 2,4-dimethoxy-6-pyrimidinyl, 4-methoxy-6-pyrimidinyl, 3-amino-2-pyrazinyl, 2-amino-5-methyl-3-pyrazinyl, 3-amino-5,6-dimethyl-2-pyrazinyl or 4,6-dimethyl-2-pyrimidinyl.

13. A herbicidal and/or plant growth-regulating composition which contains as at least one active component a compound of the formula I according to claim 1.

14. A composition according to claim 13 which contains 0.01 to 95 % by weight of a compound of the formula I according to claim 1, and 1 to 99.99 % by weight of adjuvants, including 0 to 25 % by weight of a surfactant.

15. Use of a compound of formula I according to claim 1 for controlling undesired varieties of plants in crops of useful plants and/or for regulating the growth of useful plants.

16. Use according to claim 15 for selective weed control.

17. Use according to claim 15 for inhibiting plant growth.

18. A method of controlling weeds pre- or post-emergence and/or of influencing the growth of useful plants, which method comprises applying to harmful or useful plants, to parts of said plants or to the locus thereof, an effective amount of an active ingredient of the formula I according to claim 1.

**Claims** (for the Contracting State AT)

1. Herbicidal and/or plant growth-regulating composition that contains as at least one active component a compound of the formula I

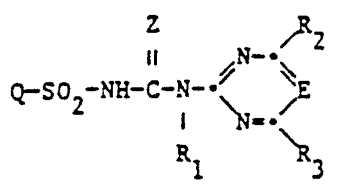

(I)

or a salt thereof, wherein

$R_1$ is hydrogen or $C_1$-$C_5$alkyl, each of

$R_2$ and $R_3$, independently of the other, is hydrogen, $C_1$-$C_5$alkyl, $C_1$-$C_5$alkoxy, $C_1$-$C_5$alkylthio, $C_1$-$C_5$haloalkyl, halogen, $C_1$-$C_5$haloalkoxy, $C_1$-$C_5$haloalkylthio, $C_1$-$C_4$alkylamino, di-$C_1$-$C_4$alkylamino, or an alkoxyalkyl group or alkoxyalkoxy group containing not more than 6 carbon atoms,

Z is oxygen or sulfur,

E is —CH= or —N=, and

Q is a pyrimidine, pyridazine, pyrazine or triazine radical that is bonded by way of a carbon atom and is unsubstituted or substituted by halogen, pseudohalogen, nitro, $C_1$-$C_6$alkyl, $C_1$-$C_6$haloalkyl, $C_1$-$C_6$alkoxy, $C_1$-$C_6$alkylthio, $C_1$-$C_6$haloalkoxy, $C_1$-$C_6$haloalkylthio, amino, $C_1$-$C_6$alkylamino, di-$C_1$-$C_6$alkylamino, $C_1$-$C_6$alkylcarbonylamino, $C_1$-$C_6$alkylcarbonyl, $C_1$-$C_6$alkoxycarbonyl, $C_1$-$C_6$alkylthiocarbonyl, carbamoyl, $C_1$-$C_6$alkylaminocarbonyl, di-$C_1$-$C_6$alkylaminocarbonyl, $C_1$-$C_6$alkylsulfinyl, $C_1$-$C_6$alkylsulfonyl, $C_2$-$C_6$alkenyloxy, $C_3$-$C_6$alkynyloxy, or by phenyl, phenoxy or phenylthio each of which is unsubstituted or substituted by halogen, nitro, cyano, $C_1$-$C_6$alkyl, $C_1$-$C_6$alkoxy, $C_1$-$C_6$haloalkyl or by $C_1$-$C_6$haloalkoxy, or by a benzyl group that is unsubstituted or substituted by halogen and/or $C_1$-$C_6$alkyl.

2. A composition according to claim 1 wherein Z is oxygen and $R_1$ is hydrogen.

3. A composition according to claim 1 wherein $R_1$ is hydrogen, each of $R_2$ and $R_3$, independently of the other, is $C_1$-$C_3$alkyl, $C_1$-$C_3$alkoxy, $C_1$-$C_3$haloalkyl, $C_1$-$C_3$haloalkoxy, halogen or $C_2$-$C_4$alkoxyalkyl, Z is oxygen, E is nitrogen or CH, and Q is a heterocyclic radical as defined for formula I that is unsubstituted or substituted by chlorine, bromine, fluorine, nitro, cyano, $C_1$-$C_4$alkylcarbonyl, $C_3$-$C_5$alkoxyalkoxy, $C_1$-$C_4$alkoxycarbonyl, $C_1$-$C_3$alkoxy, $C_1$-$C_3$alkylthio, $C_1$-$C_3$alkylsulfinyl, $C_1$-$C_3$alkylsulfonyl, $C_1$-$C_4$alkyl, trif-

luoromethyl, trichloromethyl, cyano-$C_1$-$C_4$alkyl, $C_2$-$C_4$alkenyloxy, $C_3$-$C_4$alkynyloxy or by N-$C_1$-$C_4$alkylcarbonylamino.

4. A composition according to claim 3 wherein Q is a pyrazine radical.

5. A composition according to claim 1 that contains as active ingredient N-(2-methylpyrazin-3-ylsulfonyl)-N'-(4-methoxy-6-methyl-1,3,5-triazin-2-yl)-urea.

6. A composition according to claim 1 that contains as active ingredient N-(2-methoxypyrazin-3-ylsulfonyl)-N'-(4-methoxy-6-methyl-1,3,5-triazin-2-yl)-urea.

7. A process for the preparation of compounds of the formula I, which comprises reacting a phenylsulfonyl derivative of the formula II

$$Q—SO_2W \qquad (II)$$

with a compound of the formula III

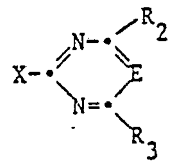

(III)

wherein X and W are one of the groups $—NH_2$, $—N=C=Z$ or $—NR_1—CZ—OR$, the substituents $R_1$, $R_2$, $R_3$, Q, Z and E are as defined for formula I, and R is an aliphatic or aromatic radical, with the proviso that the reactants of the formulae II and III are always so selected that an amino function reacts either with an isocyanato or isothiocyanato group or with an [$—NR_1—CZ—OR$] group.

8. A process according to claim 7 wherein R is $C_1$-$C_4$alkyl, phenyl or benzyl.

9. A process according to claim 7 wherein the reaction is carried out in the presence of a solvent that is inert towards the reaction or a solvent mixture.

10. A process according to claim 9 wherein the reaction is carried out in the presence of a base.

11. A composition according to claim 1 which contains 0.01 to 95 % by weight of a compound of the formula I according to claim 1, and 1 to 99.99 % by weight of adjuvants, including 0 to 25 % by weight of a surfactant.

12. Use of a compound of formula I according to claim 1 for controlling undesired varieties of plants in crops of useful plants and/or for regulating the growth of useful plants.

13. Use according to claim 12 for selective weed control.

14. Use according to claim 12 for inhibiting plant growth.

15. A method of controlling weeds pre- or post-emergence and/or of influencing the growth of useful plants, which method comprises applying to harmful or useful plants, to parts of said plants or to the locus thereof, an effective amount of an active ingredient of the formula I according to claim 1.

**Revendications** (pour les Etats contractants : BE, CH, DE, FR, GB, IT, LI, NL)

1. Composés de formule I

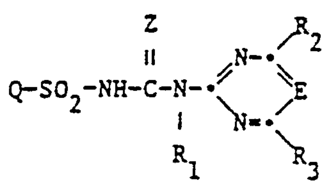

(I)

dans laquelle

$R_1$ représente l'hydrogène ou un groupe alkyle en C 1-C 5 ;

$R_2$ et $R_3$ représentent chacun, indépendamment l'un de l'autre, l'hydrogène, un groupe alkyle en C 1-C 5, alcoxy en C 1-C 5, alkylthio en C 1-C 5, halogénoalkyle en C 1-C 5, un halogène, un groupe halogénoalcoxy en C 1-C 5, halogénoalkylthio en C 1-C 5, alkylamino en C 1-C 4, di-(alkyle en C 1-C 4)-amino, un groupe alcoxyalkyle ou alcoxyalcoxy contenant au maximum 6 atomes de carbone ;

Z représente l'oxygène ou le soufre ;

E représente —CH= ou —N=, et

Q représente un radical de pyromidine, de pyridazine, de pyrazine ou de triazine relié par l'intermédiaire d'un atome de carbone et éventuellement substitué par des halogènes, des pseudo-halogènes, des groupes nitro, alkyle en C 1-C 6, halogénoalkyle en C 1-C 6, alcoxy en C 1-C 6, alkylthio en C 1-C 6, halogénoalcoxy en C 1-C 6, halogénoalkylthio en C1-C6, amino, alkylamino en C 1-C 6, di-(alkyle en C 1-C 6)-amino, (alkyle en C 1-C 6)-carbonylamino, (alkyle en C 1-C 6)-carbonyle, (alcoxy en C 1-C 6)-carbonyle, (alkyle en C 1-C 6)-thiocarbonyle, carbamoyle, (alkyle en C 1-C 6)-aminocarbonyle, di(alkyle en

C 1-C 6)-aminocarbonyle, alkylsulfinyle en C 1-C 6, alkylsulfonyle en C 1-C 6, alcényloxy en C 2-C 6, alcynyloxy en C 3-C 6 ou par un groupe phényle, phénoxy, phénylthio éventuellement substitué par des halogènes, des groupes nitro, cyano, alkyle en C 1-C 6, alcoxy en C 1-C 6, halogénoalkyle en C 1-C 6, halogéno-alcoxy en C 1-C 6 ou par un groupe benzyle éventuellement substitué par des halogènes et/ou des groupes alkyle en C 1-C 6, et les sels de ces composés.

2. Composés de formule I selon la revendication 1, caractérisés en ce que Z représente l'oxygène et $R_1$ l'hydrogène.

3. Composés de formule I selon la revendication 1, caractérisés en ce que $R_1$ représente l'hydrogène, $R_2$ et $R_3$ représentent chacun, indépendamment l'un de l'autre, un groupe alkyle en C 1-C 3, alcoxy en C 1-C 3, halogénoalkyle en C 1-C 3, halogénoalcoxy en C 1-C 3, un halogène ou un groupe alcoxyalkyle en C 2-C 4, Z représente l'oxygène, E représente l'azote ou CH, Q représente un radical hétérocyclique tel que défini en référence à la formule I, éventuellement substitué par le chlore, le brome, le fluor, des groupes nitro, cyano, (alkyle en C 1-C 4)-carbonyle, alcoxyalcoxy en C 3-C 5, (alkyle en C 1-C 4)-oxycarbonyle, alcoxy en C 1-C 3, alkylthio en C 1-C 3, alkylsulfinyle en C 1-C 3, alkylsulfonyle en C 1-C 3, alkyle en C 1-C 4, trifluorométhyle, trichlorométhyle, cyano-(alkyle en C 1-C 4), alcényloxy en C 2-C 4, alcynyloxy en C 3-C 4 ou N-(alkyle en C 1-C 4)-carbonylamino.

4. Composés selon la revendication 3, caractérisés en ce que Q représente un radical pyrazinyle.

5. La N-(2-méthyl-pyrazine-3-ylsulfonyl)-N'-(4-méthoxy-6-méthyl-1,3,5-triazine-2-yl)-urée selon la revendication 1.

6. La N-(2-méthoxy-pyrazine-3-ylsulfonyl)-N'-(4-méthoxy-6-méthyl-1,3,5-triazine-2-yl)-urée selon la revendication 1.

7. Procédé de préparation des composés de formule I, caractérisé en ce que l'on fait réagir un dérivé phénylsulfonylique de formule II

$$Q—SO_2W \qquad (II)$$

avec un composé de formule III

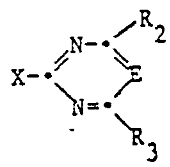

(III)

dans lesquelles X et W représentent l'un des groupes $—NH_2$, $—N=C=Z$ ou $—NR_1—CZ—OR$ ; les symboles $R_1$, $R_2$, $R_3$, Q, Z et E ont les significations indiquées en référence à la formule I, R représente un radical aliphatique ou aromatique ; sous réserve que les réactifs de formules II et III sont toujours choisis de manière qu'une fonction amino réagisse soit avec un groupe isocyanato ou isothiocyanato, soit avec un groupe $—NR_1—CZ—CR$.

8. Procédé selon la revendication 7, caractérisé en ce que R représente un groupe alkyle en C 1-C 4, phényle ou benzyle.

9. Procédé selon la revendication 7, caractérisé en ce que l'on opère en présence d'un solvant ou mélange solvant inerte dans la réaction.

10. Procédé selon la revendication 9, caractérisé en ce que la réaction est effectuée en présence d'une base.

11. Composés de formule

$$Q—SO_2—NH—CZ—OR$$

dans laquelle Q et Z ont les significations indiquées en référence à la formule I dans la revendication 1 et R a les significations indiquées en référence à la formule II dans la revendication 7.

12. Composés de formule

$$Q—SO_2—NH_2$$

dans laquelle Q a les significations indiquées en référence à la formule I dans la revendication 1, sauf les suivantes : 4-méthyl-2-pyrimidinyle, 5-méthyl-2-pyrimidinyle, 2,4-diméthoxy-6-pyrimidinyle, 4-méthoxy-6-pyrimidinyle, 3-amino-2-pyrazinyle, 2-amino-5-méthyl-3-pyrazinyle, 3-amino-5,6-diméthyl-2-pyrazinyle ou 4,6-diméthyl-2-pyrimidinyle.

13. Produit herbicide et/ou régulateur de croissance des végétaux, caractérisé en ce qu'il contient au moins un composant actif consistant en un composé de formule I selon la revendication 1.

14. Produit selon la revendication 13, caractérisé en ce qu'il contient de 0,01 à 95 % en poids d'un

composé de formule I selon la revendication 1.et de 1 à 99,99 % en poids d'additifs parmi lesquels 0 à 25 % en poids d'un agent tensioactif.

15. Utilisation d'un composé de formule I selon la revendication 1 pour combattre les végétaux indésirables dans les cultures de végétaux utiles et/ou pour la régulation de la croissance des végétaux utiles.

16. Utilisation selon la revendication 15, pour la lutte sélective contre les mauvaises herbes.

17. Utilisation selon la revendication 15, pour l'inhibition de la croissance des végétaux.

18. Procédé pour combattre en pré- ou post-levée les végétaux adventices et/ou pour agir sur la croissance des végétaux utiles, caractérisé en ce que l'on applique une quantité efficace d'une substance active de formule I selon la revendication 1 sur la plante nuisible ou utile, des parties de cette plante ou son habitat.

**Revendications** (pour l'Etat contractant AT)

1. Produit herbicide et/ou régulateur de la croissance des végétaux, caractérisé en ce qu'il contient au moins un composant actif consistant en un composé de formule I

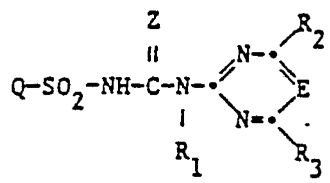

(I)

ou un sel d'un tel composé, dans lesquels

$R_1$ représente l'hydrogène ou un groupe alkyle en C 1-C 5 ;

$R_2$ et $R_3$ représentent chacun, indépendamment l'un de l'autre, l'hydrogène, un groupe alkyle en C 1-C 5, alcoxy en C 1-C 5, alkylthio en C 1-C 5, halogénoalkyle en C 1-C 5, un halogène, un groupe halogénoalcoxy en C 1-C 5, halogénoalkylthio en C 1-C 5, alkylamino en C 1-C 4, di-(alkyle en C 1-C 4)-amino, un groupe alcoxyalkyle ou alcoxyalcoxy contenant au maximum 6 atomes de carbone ;

Z représente l'oxygène ou le soufre ;

E représente —CH= ou —N=, et

Q représente un radical de pyrimidine, de pyridazine, de pyrazine ou de triazine relié par l'intermédiaire d'un atome de carbone, éventuellement substitué par des halogènes, des pseudohalogènes, des groupes nitro, alkyle en C 1-C 6, halogénoalkyle en C 1-C 6, alcoxy en C 1-C 6, alkylthio en C 1-C 6, halogénoalcoxy en C 1-C 6, halogénoalkylthio en C 1-C 6, amino, alkylamino en C 1-C 6, di-(alkyle en C 1-C 6)-amino, (alkyle en C 1-C 6)-carbonylamino, (alkyle en C 1-C 6)-carbonyle, (alcoxy en C 1-C 6)-carbonyle, (alkyle en C 1-C 6)-thiocarbonyle, carbamoyle, (alkyle en C 1-C 6)-aminocarbonyle, di-(alkyle en C 1-C 6)-aminocarbonyle, alkylsulfinyle en C 1-C 6, alkylsulfonyle en C 1-C 6, alcényloxy en C 2-C 6, alcynyloxy en C 3-C 6 ou par un groupe phényle, phénoxy, phénylthio éventuellement substitué par des halogènes, des groupes nitro, cyano, alkyle en C 1-C 6, alcoxy en C 1-C 6, halogénoalkyle en C 1-C 6, halogénoalcoxy en C 1-C 6, ou par un groupe benzyle éventuellement substitué par des halogènes et/ou des groupes alkyle en C 1-C 6.

2. Produit selon la revendication 1, caractérisé en ce que Z représente l'oxygène et $R_1$ l'hydrogène.

3. Produit selon la revendication 1, caractérisé en ce que $R_1$ représente l'hydrogène, $R_2$ et $R_3$ représentent chacun, indépendamment l'un de l'autre, un groupe alkyle en C 1-C 3, alcoxy en C 1-C 3, halogénoalkyle en C 1-C 3, halogénoalcoxy en C 1-C 3, un halogène ou un groupe alcoxyalkyle en C 2-C 4, Z représente l'oxygène, E représente l'azote ou CH, Q représente un radical hétérocyclique tel que défini en référence à la formule I, éventuellement substitué par le chlore, le brome, le fluor, des groupes nitro, cyano, (alkyle en C 1-C 4)-carbonyle, alcoxyalcoxy en C 3-C 5, (alkyle en C 1-C 4)-oxycarbonyle, alcoxy en C 1-C 3, alkylthio en C 1-C 3, alkylsulfinyle en C 1-C 3, alkylsulfonyle en C 1-C 3, alkyle en C 1-C 4, trifluorométhyle, trichlorométhyle, cyano-(alkyle en C 1-C 4), alcényloxy en C 2-C 4, alcynyloxy en C 3-C 4 ou N-(alkyle en C 1-C 4)-carbonylamino.

4. Produit selon la revendication 3, caractérisé en ce que Q représente un radical de pyrazine.

5. Produit selon la revendication 1, caractérisé en ce qu'il contient en tant que substance active la N-(2-méthyl-pyrazine-3-ylsulfonyl)-N'-(4-méthoxy-6-méthyl-1,3,5-triazine-2-yl)-urée.

6. Produit selon la revendication 1, caractérisé en ce qu'il contient en tant que substance active la N-(2-méthoxy-pyrazine-3-ylsulfonyl)-N'-(4-méthoxy-6-méthyl-1,3,5-triazine-2-yl)-urée.

7. Procédé de préparation des composés de formule I, caractérisé en ce que l'on fait réagir un dérivé phénylsulfonylique de formule II

$$Q—SO_2W \qquad (II)$$

avec un composé de formule III

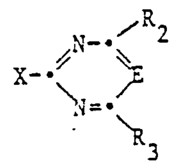

(III)

dans lesquelles X et W représentent l'un des groupes $—NH_2$, —N=C=Z ou $—NR_1—CZ—OR$ ; les symboles $R_1$, $R_2$, $R_3$, Q, Z et E ont les significations indiquées en référence à la formule I, R représente un radical aliphatique ou aromatique ; sous réserve que les réactifs de formules II et III sont toujours choisis de manière qu'une fonction amino réagisse soit avec un groupe isocyanato ou isothiocyanato, soit avec un groupe $—NR_1—CZ—OR$.

8. Procédé selon la revendication 7, caractérisé en ce que R représente un groupe alkyle en C 1-C 4, phényle ou benzyle.

9. Procédé selon la revendication 7, caractérisé en ce que l'on opère en présence d'un solvant ou mélange solvant inerte dans la réaction.

10. Procédé selon la revendication 9, caractérisé en ce que la réaction est effectuée en présence d'une base.

11. Produit selon la revendication 1, caractérisé en ce qu'il contient de 0,01 à 95 % en poids d'un composé de formule I selon la revendication 1, et de 1 à 99,99 % en poids d'additifs parmi lesquels 0 à 25 % en poids d'un agent tensioactif.

12. Utilisation d'un composé de formule I selon la revendication 1 dans la lutte contre les végétaux indésirables dans les cultures de végétaux utiles et/ou pour la régulation de la croissance des végétaux utiles.

13. Utilisation selon la revendication 12, dans la lutte sélective contre les mauvaises herbes.

14. Utilisation selon la revendication 12, pour l'inhibition de la croissance des végétaux.

15. Procédé pour combattre les mauvaises herbes en pré- ou post-levée et/ou pour agir sur la croissance des végétaux utiles, caractérisé en ce que l'on applique une quantité efficace d'une substance active de formule I selon la revendication 1 sur la plante nuisible ou utile ; des parties de cette plante ou son habitat.